# EUROPEAN PATENT APPLICATION

(11) **EP 4 830 756 A1**
(43) Date of publication of application: **28.10.2026**
(21) Application number: 26174581.4
(22) Date of filing: 24.04.2026
(51) Int. Cl.: A61M 25/06, A61M 29/00, A61M 25/00

(54) **INTRODUCER-DILATOR POSITIONING ADJUSTMENT SYSTEM**

(30) Priority: 25.04.2025 US 202563794379 P; 16.04.2026 US 202664041072 P; 19.04.2026 US 202619651724
(71) Applicant: Medtronic, Inc., Minneapolis, Minnesota 55432 (US)
(72) Inventor: McGUINN, Alan T., Minneapolis, 55432 (US); HIGGINS, Craig M., Minneapolis, 55432 (US)
(74) Representative: Zimmermann & Partner Patentanwälte mbB

(57) **Abstract**

An introducer system (100) includes an introducer (110), a dilator (130), and a coupling arrangement. The introducer includes an introducer hub (118), an introducer sheath (120), and an introducer tip. The dilator includes a dilator hub (140) and a dilator shaft (138). The coupling arrangement is configured to selectively permit rotation of the dilator relative to the introducer to longitudinally translate the dilator relative to the introducer.

## Description

### CROSS-REFERENCE TO RELATED APPLICATIONS

This application claims the benefit of the filing date of U.S. Provisional Application No. 63/794,379, filed April 25, 2025, U.S. Provisional Application No. 64/041,072, filed April 16, 2026, and U.S. Patent Application No. 19/651,724, filed April 19, 2026, the contents of which are incorporated by reference herein in their entirety.

### FIELD OF THE INVENTION

The present invention relates to an introducer system for providing percutaneous access to a patient's vasculature for a transcatheter device delivery system.

### BACKGROUND

An introducer system is used to provide percutaneous access to the vascular system of a patient and functions to permit the introduction and positioning of various minimally invasive delivery devices within the patient's vasculature. In general, making the smallest incision is the most desirable and leads to less complications, less trauma, and improved patient outcomes.

An introducer system may generally include an introducer and a dilator. The dilator is inserted within the introducer to provide axial stiffness to the introducer during initial insertion into the vessel. The dilator is then removed from the introducer to enable other devices to be advanced through the introducer, such as a delivery catheter.

The transition interface between a sheath tip of the introducer and the dilator requires a tight longitudinal length tolerance to prevent presenting a poor interface between the two components. A gap or bump presented by the transition between the introducer sheath tip and the distal portion of the dilator may increase insertion forces and may cause trauma to the patient during insertion and tracking through the vasculature of the patient. Thus, it is desirable to adjust and optimize the transition interface between the sheath tip and distal portion of the dilator to reduce the occurrence of such undesirable events.

### BRIEF SUMMARY

In accordance with an example hereof, the present disclosure relates to an introducer system. The introducer system includes an introducer, a dilator, and a coupling arrangement. The introducer includes an introducer hub, an introducer sheath, and an introducer tip. The introducer includes a proximal end, a distal end, and a central lumen extending from the proximal end to the distal end of the introducer. The dilator includes a dilator hub and a dilator shaft. The coupling arrangement is configured to couple the dilator to the introducer and is configured to selectively enable rotation of the dilator relative to the introducer to longitudinally translate the dilator relative to the introducer.

In accordance with another example hereof, in the introducer system of any of the preceding or following examples, the coupling arrangement is a coupling ring.

In accordance with another example hereof, in the introducer system of any of the preceding or following examples, the coupling ring is coupled to the dilator of the introducer system.

In accordance with another example hereof, in the introducer system of any of the preceding or following examples, the coupling ring is coupled to the introducer of the introducer system.

In accordance with another example hereof, in the introducer system of any of the preceding or following examples, the coupling ring comprises a distal section configured to couple the coupling ring to the introducer hub.

In accordance with another example hereof, in the introducer system of any of the preceding or following examples, the dilator hub comprises a distal portion including a first thread on an outer surface of the distal portion, and the coupling ring comprises a middle section including a second thread on an inner surface of the middle section. The first thread of the distal portion of the dilator hub threadably engages the second thread of the second section of the coupling ring to enable longitudinal translation of the dilator relative to the coupling ring when the dilator hub is rotated relative to the coupling ring.

In accordance with another example hereof, in the introducer system of any of the preceding or following examples, the introducer system includes a first configuration wherein the coupling ring is coupled to the introducer hub, the dilator is disposed through the introducer, a distal end of the distal hub abuts a distal end of the distal end of the second section of the coupling ring, and a distal end of the dilator shaft extends distally past the introducer tip. Rotation of the dilator hub in a first direction relative to the coupling ring longitudinally translates the dilator proximately relative to the introducer to a second configuration.

In accordance with another example hereof, in the introducer system of any of the preceding or following examples, the introducer system in the first configuration includes the introducer tip in an undercaptured configuration relative to the dilator shaft and in the second configuration the introducer tip is aligned in an aligned configuration relative to the dilator shaft.

In accordance with another example hereof, in the introducer system of any of the preceding or following examples, further comprising a lock assembly configured to selectively transition the dilator and the coupling ring between a locked configuration in which the dilator hub is prevented from rotating relative to the coupling ring and an unlocked configuration in which the dilator hub is enabled to rotate relative to the coupling ring.

In accordance with another example hereof, in the introducer system of any of the preceding or following examples, the lock assembly comprises a tab including a first and a second end, an actuator coupled to the second end of the tab and configured to be selectively actuated by a user to transition the lock assembly between the locked configuration and the unlocked configuration, and a biasing element configured to urge the lock assembly to the locked configuration.

In accordance with another example hereof, in the introducer system of any of the preceding or following examples, the coupling ring comprises a plurality of slots. When the lock assembly is in the locked configuration the tab of the lock assembly is disposed within one of the plurality of slots to prevent rotation of the dilator hub relative to the coupling ring. When the lock assembly is in the unlocked configuration, the tab is not disposed in any of the plurality of slots such that the dilator hub is free to rotate relative to the coupling ring.

In accordance with another example hereof, in the introducer system of any of the preceding or following examples: the dilator hub comprises an outer body and an inner body disposed within the outer body, wherein the outer body is rotatably coupled to the inner body such that rotation of the outer body does not rotate the inner body and longitudinal translation of the outer body causes longitudinal translation of the inner body, wherein the dilator shaft is coupled to the inner body such that movement of the inner body causes corresponding movement of the dilator shaft; the introducer hub comprises a proximal extension extending proximally from a body of the introducer hub; and the coupling arrangement comprises a first thread and a second thread coupling the outer body to the proximal extension, wherein the first thread and the second thread convert rotation of the outer body to longitudinal translation of the dilator hub relative to the introducer hub, and hence longitudinal translation of the dilator shaft relative to the introducer sheath.

In accordance with another example hereof, in the introducer system of any of the preceding or following examples: the proximal extension is a cylindrical extension defining a portion of a central lumen of the introducer, wherein the proximal extension includes the first thread on an outer surface thereof; and the outer body includes a distal portion, wherein the second thread is disposed on an inner surface of the distal portion.

In accordance with another example hereof, in the introducer system of any of the preceding or following examples, the outer body includes a tab extending radially inward and circumferentially around an inner surface of the outer body, and wherein the inner body includes a recess extending radially inward and circumferentially around an outer surface of the inner body, wherein the tab and the recess rotatably couple the outer body to the inner body.

In accordance with another example hereof, the introducer system of any of the preceding or following examples further comprises a lock configured to lock the relative positions of the introducer and the dilator.

In accordance with another example hereof, in the introducer system of any of the preceding or following examples, the lock comprises a slider disposed on the introducer hub and a ridged distal end of the outer body of the dilator hub, wherein the slider comprises a shaped end, wherein in an unlocked configuration, the shaped end of the slider is spaced from the ridged distal end of the outer body, and wherein in a locked configuration, the shaped end of the slider engages the ridged distal end of the outer body.

In accordance with another example hereof, in the introducer system of any of the preceding or following examples, the coupling arrangement comprises tabs of the dilator hub interacting with a stepped channel of the introducer hub such that rotation of the dilator hub converts to longitudinal translation of the dilator relative to the introducer.

In accordance with another example hereof, in the introducer system of any of the preceding or following examples: the dilator hub comprises an outer body and an inner body disposed within the outer body, wherein the outer body is rotatably coupled to the inner body such that rotation of the outer body does not rotate the inner body and longitudinal translation of the outer body causes longitudinal translation of the inner body, wherein the dilator shaft is coupled to the inner body such that movement of the inner body causes corresponding movement of the dilator shaft, wherein the dilator hub comprises tabs at a distal end thereof; and the tabs extend radially outward from a distal portion of the outer body.

In accordance with another example hereof, the introducer system of any of the preceding or following examples further comprises a lock configured to lock the relative positions of the introducer and the dilator.

In accordance with another example hereof, in the introducer system of any of the preceding or following examples, the lock comprises a spring disposed within the introducer hub, wherein the spring is configured to act on the outer body of the dilator hub to press the tabs against a wall of the stepped channel to prevent relative movement between the dilator and the introducer.

In accordance with an example hereof, a method of adjusting a relative longitudinal position of an introducer and a dilator of an introducer system comprises releasably coupling a dilator to a coupling ring and setting the dilator and the coupling ring to a locked configuration, disposing the dilator through the introducer such that the coupling ring is releasably coupled to the introducer and a distal portion of a dilator shaft of the dilator extends distally past and introducer tip of the introducer in an under captured configuration, transitioning the dilator and the coupling ring to an unlocked configuration, rotating the dilator relative to the coupling ring to longitudinally translate the dilator relative to the introducer, and transitioning the dilator and the coupling ring to the locked configuration with the introducer system in an aligned configuration.

In accordance with another example hereof, in the method of any of the preceding or following examples, the dilator is rotated relative to the coupling ring to longitudinally translate the dilator relative to the introducer until a transition interface between the introducer tip is in the aligned configuration.

In accordance with another example hereof, in the method of any of the preceding or following examples, setting the dilator and the coupling ring to the locked configuration comprises transitioning a lock assembly to a first state to prevent rotation of the dilator relative to the coupling ring.

In accordance with another example hereof, in the method of any of the preceding or following examples, transitioning the dilator and the coupling ring to the unlocked configuration comprises pushing an actuator of a lock assembly such that a tab of the lock assembly exits a slot of the coupling ring to enable the dilator to rotate relative to the coupling ring.

In accordance with another example hereof, in the method of any of the preceding or following examples, transitioning the dilator and the coupling ring to the locked configuration comprises releasing the actuator such that a biasing element of the lock assembly returns the tab to a slot of the coupling ring such that the tab is disposed within the slot and prevents rotation of the dilator relative to the coupling ring.

In accordance with another example hereof, in the method of any of the preceding or following examples, coupling the dilator to the coupling ring comprises rotating dilator relative to the coupling ring such that a first thread on an outer surface of a dilator hub of the dilator engages a second thread on an inner surface of the coupling ring.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, serve to explain the principles of the present disclosure and to enable a person skilled in the pertinent art to make and use the embodiments of the present disclosure. The drawings may not be to scale.
FIG. 1 depicts a perspective side view of an introducer system with a coupling ring, according to embodiments hereof.
FIG. 2 is a perspective view of an introducer of the introducer system of FIG. 1, according to embodiments hereof.
FIG. 3 is a perspective view of a dilator of the introducer system of FIG. 1, according to embodiments hereof.
FIG. 4 depicts an enlarged perspective view of the coupling ring and a portion of the dilator of the introducer system of FIG. 1, according to embodiments hereof.
FIG. 5 depicts an enlarged side view of a distal portion of a dilator hub of the introducer system of FIG. 1, according to embodiments hereof.
FIG. 6 depicts an enlarged perspective view of the distal portion of the dilator hub of the introducer system of FIG. 1, according to embodiments hereof.
FIG. 7 depicts an enlarged side view of the coupling ring of the introducer system of FIG. 1, according to embodiments hereof.
FIG. 8 depicts an enlarged perspective view of the coupling ring of the introducer system of FIG. 1, according to embodiments hereof.
FIG. 9 is a flow chart depicting a method of adjusting the longitudinal position of a dilator relative to an introducer, according to embodiments hereof.
FIGS. 10-15 depict illustrations of several of the steps of the method of FIG. 9 of adjusting the longitudinal position of a dilator relative to an introducer.
FIG. 16 depicts a cross-sectional illustration of an introducer system according to embodiments hereof.
FIG. 17 depicts a cross-sectional illustration of the introducer system of FIG. 16 with a dilator longitudinally advanced relative to an introducer.
FIG. 18 depicts a side view illustration of the introducer system of FIG. 16 with a lock in an unlocked configuration.
FIG. 19 depicts a side view illustration of the introducer system of FIG. 16 with the lock of FIG. 18 in a locked configuration.
FIG. 20 depicts a cross-sectional illustration of an introducer system according to embodiments hereof.
FIG. 21 depicts a cross-sectional illustration of the introducer system of FIG. 20 with a dilator coupled to an introducer.
FIG. 22 depicts a close-up illustration of a portion of the introducer and a portion of the dilator of the introducer system of FIG. 20.
FIG. 23 depicts a close-up view illustration of a portion of the introducer and a portion of the dilator of the introducer system of FIG. 20 with the dilator hub engaged with the introducer hub.
FIG. 24 depicts a cross-sectional view illustration of a portion of the introducer and a portion of the dilator of the introducer system of FIG. 20 showing a spring disposed within the introducer hub.
FIGS. 25A-25E shows steps in a method of coupling together the introducer hub and the dilator hub of the introducer system of FIG. 20 and adjusting the relative longitudinal position of the dilator relative to the introducer.
FIG. 26 shows a cross-sectional view illustration of a distal end of the dilator shaft and a distal end of an introducer sheath of the introducer system of FIG. 16 or the introducer system of FIG. 20.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a delivery device. The following detailed description is merely exemplary in nature and is not intended to limit the invention of the application and uses of the invention. Furthermore, there is no intention to be bound by any expressed or implied theory presented in the preceding field of the invention, background, summary, or the following detailed description.

As used in this specification, the singular forms "a", "an" and "the" specifically also encompass the plural forms of the terms to which they refer, unless the content clearly dictates otherwise.

The terms "proximal" and "distal" herein are used with reference to the clinician using the devices. Therefore, "proximal" and "proximally" mean in the direction toward the clinician, and "distal" and "distally" mean in the direction away from the clinician. In other words, "proximal" and "proximally" mean towards the hub end of the introducer and/or dilator and "distal" and "distally" means toward the tip end of the introducer and/or dilator.

Embodiments disclosed herein are directed to introducer systems configured to enable the precise and fine adjustment of a transition interface between an introducer and a dilator of an introducer system. The introducer systems are configured to permit fine adjustment of and optimization of the transition interface between the introducer and the dilator by rotationally moving the dilator relative to the introducer to longitudinally move a distal end of the dilator relative to a distal end of an introducer sheath a small, incremental distance relative to the length of the introducer to decrease introducer system insertion forces and prevent harm to a patient.

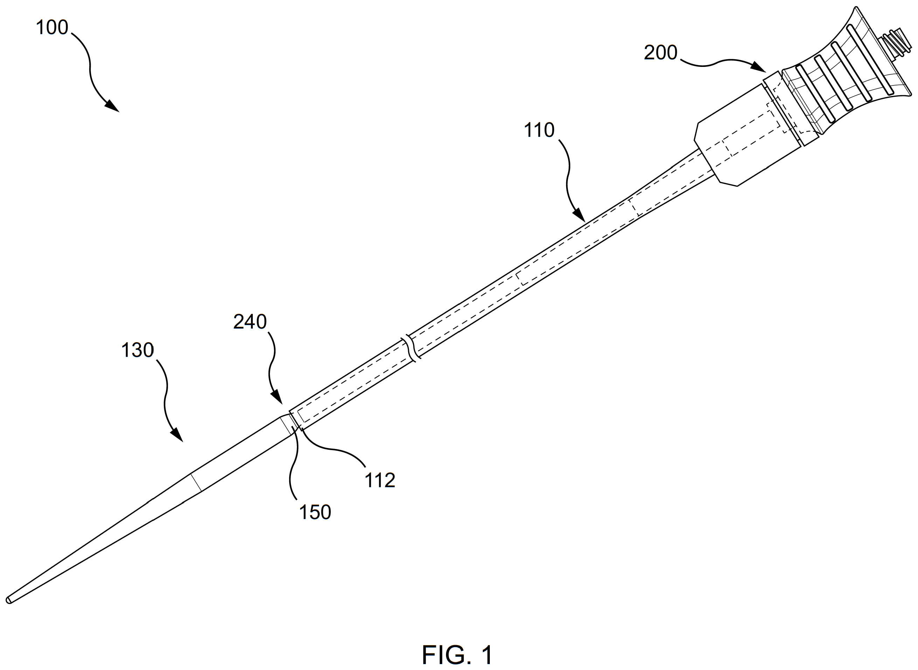

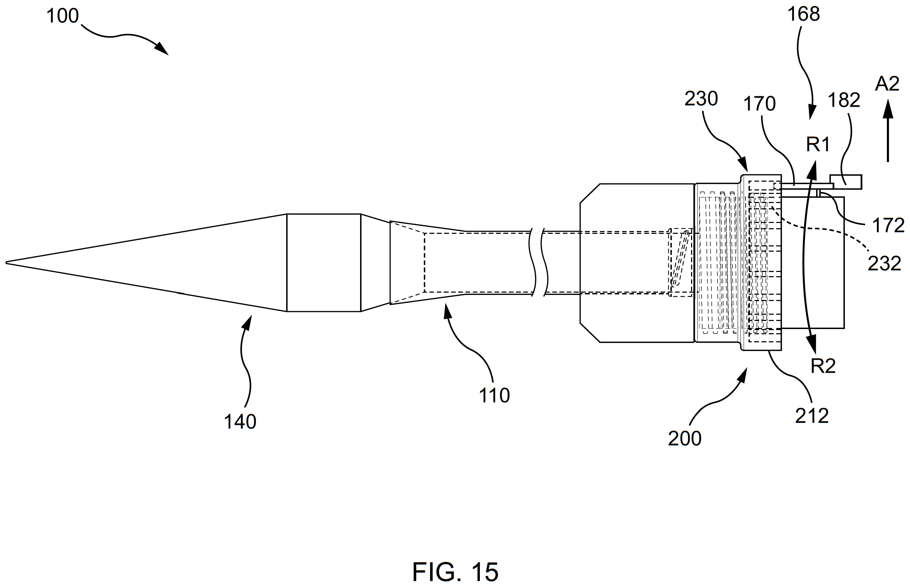

FIGS. 1-15 show an introducer system 100 according to embodiments herein. The introducer system 100 includes an introducer 110, a dilator 130, and a coupling ring 200. The introducer system 100 is configured to provide a stable central vascular access point and to stretch or expand the vasculature to permit insertion and manipulation of catheters, guidewires, or other medical instruments requiring vascular access. The coupling ring 200 is configured to enable adjustment of the interface between the introducer 110 and the dilator 130 of the introducer system 100, as described below.

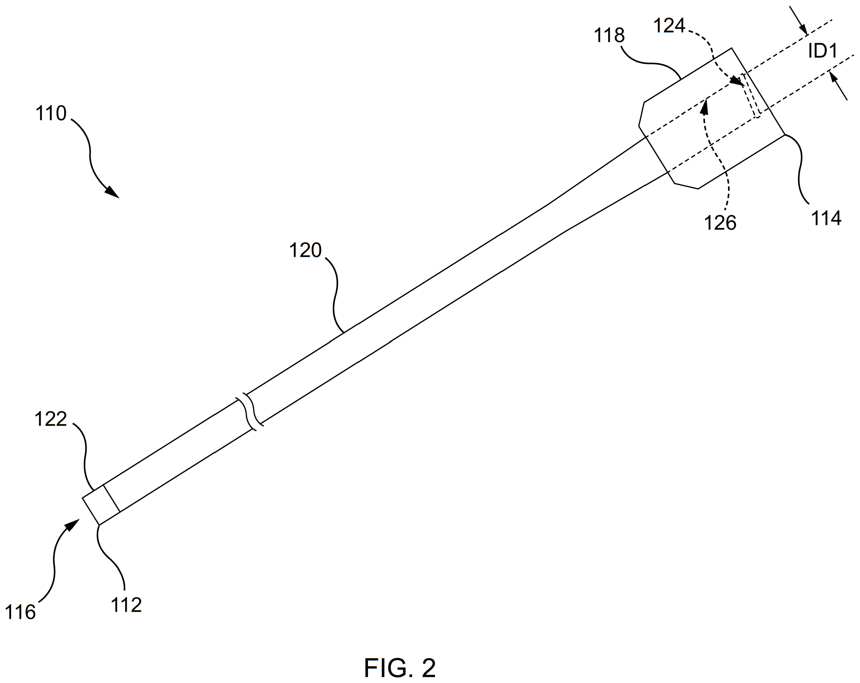

The introducer 110 includes a first or distal end 112, a second or proximal end 114, and a central lumen 116 extending from the first end 112 to the second end 114, as shown in FIG. 2. The central lumen 116 of the introducer 110 is configured to receive a dilator shaft of the dilator 130 for initial access to the vessel of the vasculature, and is configured to receive other medical devices therein after removal of the dilator 130 to provide access to a desired location within the vasculature of a patient. The introducer 110 includes an introducer hub 118, a sheath 120, and a distal tip 122. The distal tip 122 is coupled to and covers a distal end of the sheath 120. A proximal end of the sheath 120 is coupled to a distal end of the introducer hub 118. Though not shown in FIG. 2, the introducer 110 may include additional components as described in International Patent Publication No. WO 2023/147422 to Medtronic, Inc., which is incorporated by reference herein in its entirety.

The sheath 120 of FIGS. 1 and 2 may be of an elastic or expanding sheath as described, for example, in U.S. Published Application Publication No. 2014/0236122, and/or International Patent Publication No. WO 2025/003844 to Medtronic, Inc., each of which are incorporated by reference herein in their entireties. The sheath 120 generally includes a flexible, radially expandable shaft extending from the introducer hub 118 to the distal tip 122. The sheath 120 defines a distal portion of the central lumen 116 of the introducer 110.

The introducer hub 118 of FIGS. 1 and 2 may be similar to introducer hubs described in International Patent Publication No. WO 2023/147422 and/or International Patent Publication No. WO 2025/003844 to Medtronic, Inc., each of which are incorporated by reference herein in their entireties. The introducer hub 118 includes a proximal portion of the central lumen 116 of the introducer 110. In embodiments, a proximal portion of the central lumen 116 is configured to receive a distal portion of the coupling ring 200, as described below. The central lumen 116 includes a first inner diameter ID1 at the second end 114 of the introducer hub 118. In the embodiments of FIGS. 1-15, the introducer hub 118 includes a first coupling thread 124 extending radially inward from an inner surface 126 of a proximal portion of the introducer hub 118. The first coupling thread 124 is configured to engage a corresponding thread of the coupling ring 200 as described below. The first coupling thread 124 may be formed for example, and not by way of limitation, as an integral section of the introducer hub 118, or as a separate unit coupled to introducer hub 118 by any suitable method such as, but not limited to adhesives, welding, or other methods as appropriate. While the first coupling thread 124 of the introducer hub 118 is shown in FIG. 2 with a specific gender, thread form, thread angle, lead, pitch, and start, this is not meant to limit the design and other configurations and combinations may be utilized.

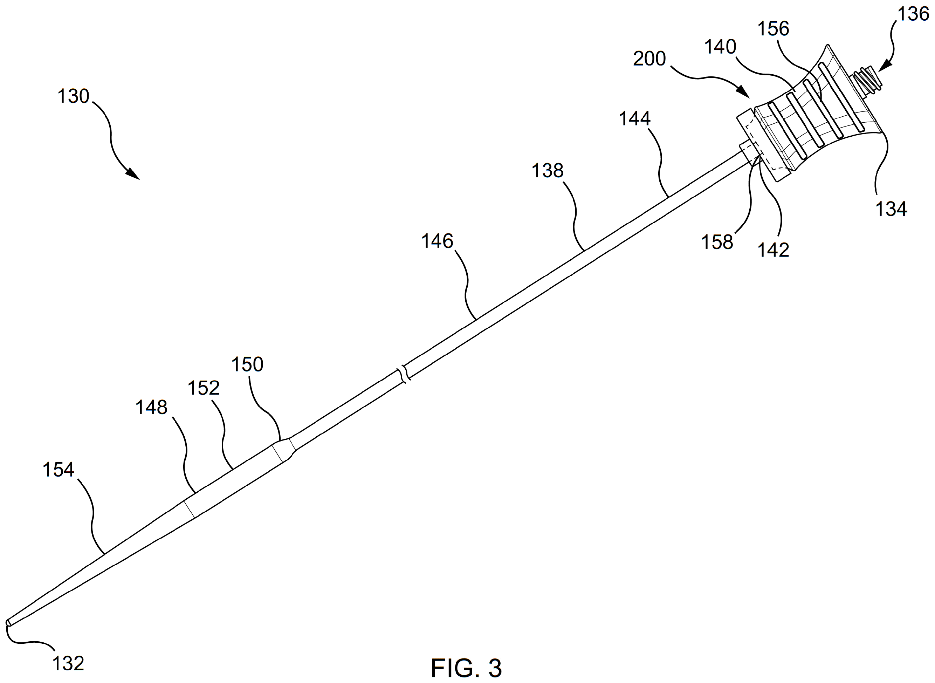

FIGS. 1 and 3 show the dilator 130 with the coupling ring 200 coupled thereto, according to embodiments hereof. The dilator 130 is configured to be used with an expandable introducer, such as, but not limited to the introducer 110 described herein. However, this is not meant to be limiting, and the dilator 130 may be used with other expandable introducers and other expandable sheaths. Those skilled in the art will recognize that features may be added and/or removed from the dilator 130.

As shown in FIG. 3, the dilator 130 includes a first or distal end 132, a second or proximal end 134, and a central lumen 136 extending from the first end 132 to the second end 134. The dilator 130 includes a dilator shaft 138 coupled to and extending distally from a dilator hub 140.

The dilator shaft 138 includes a first or distal end that defines the first or distal end 132 of the dilator 130 and a second or proximal end 142 coupled to the dilator hub 140. The dilator shaft 138 defines a distal portion of the central lumen 136 of the dilator 130. The dilator shaft 138 further includes a proximal portion 144, a middle portion 146, and a distal portion 148. The proximal portion 144 of the dilator shaft 138 is configured to be received within the introducer hub such as the introducer hub 118 described above. The middle portion 146 of the dilator shaft 138 has a reduced outer diameter as compared to the proximal portion 144. The distal portion 148 of the dilator shaft 138 extends distally from the middle portion 146. The distal portion 148 may include a distal transition portion 150, a distal cylindrical portion 152, and a distal tapered tip 154. In the embodiment shown, the distal transition portion 150 extends from the middle portion 146 and flares radially outwardly. The distal cylindrical portion 152 extends distally from a distal end of the distal transition portion. The distal tapered tip 154 extends distally from a distal end of the distal cylindrical portion 152 and tapers in a distal direction to the first or distal end 132 of the dilator 130. The distal tapered tip 154 is configured for atraumatic insertion of the dilator shaft 138 into a vessel.

The dilator hub 140 includes a body or housing 156 including a first or distal end 158, a second or proximal end that defines the second or proximal end 134 of the dilator 130, and a portion of the central lumen 136 of the dilator 130. The housing 156 provides a surface for convenient handling and grasping and manipulating by a user and may have a generally cylindrical shape as shown.

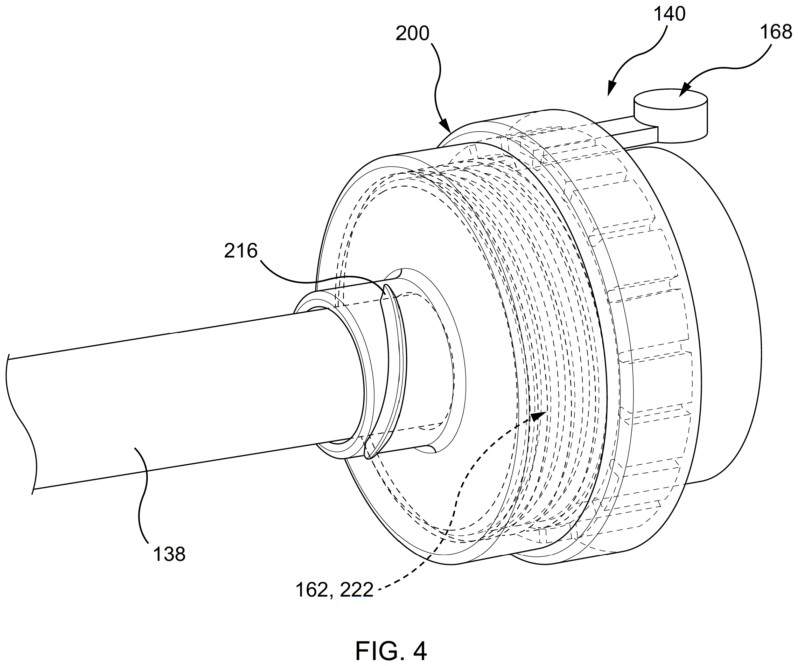

FIGS. 4-6 illustrate a portion of the dilator hub 140. In embodiments herein, the dilator hub 140 includes a first adjusting thread 162 on a distal portion 164 thereof. In an embodiment, the first adjusting thread 162 extends radially outward from an outer surface 166 of the distal portion 164. The first adjusting thread 162 is configured to engage a corresponding thread of the coupling ring 200 as described below. The first adjusting thread 162 may be formed for example, and not by way of limitation, as an integral section of the dilator hub 140, or as a separate unit coupled to the distal portion 164 of the dilator hub 140 by any suitable method such as, but not limited to adhesives, welding, or other methods as appropriate. While the first adjusting thread 162 of the dilator hub 140 is shown in FIGS. 4-6 with a specific gender, thread form, thread angle, lead, pitch, and start, this is not meant to limit the design and other configurations and combinations may be utilized.

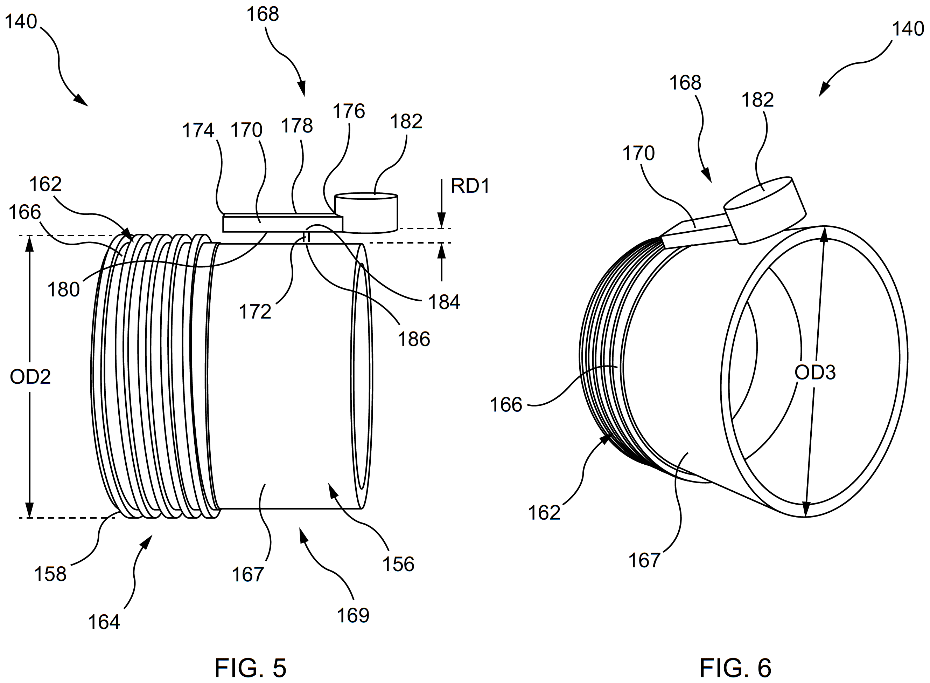

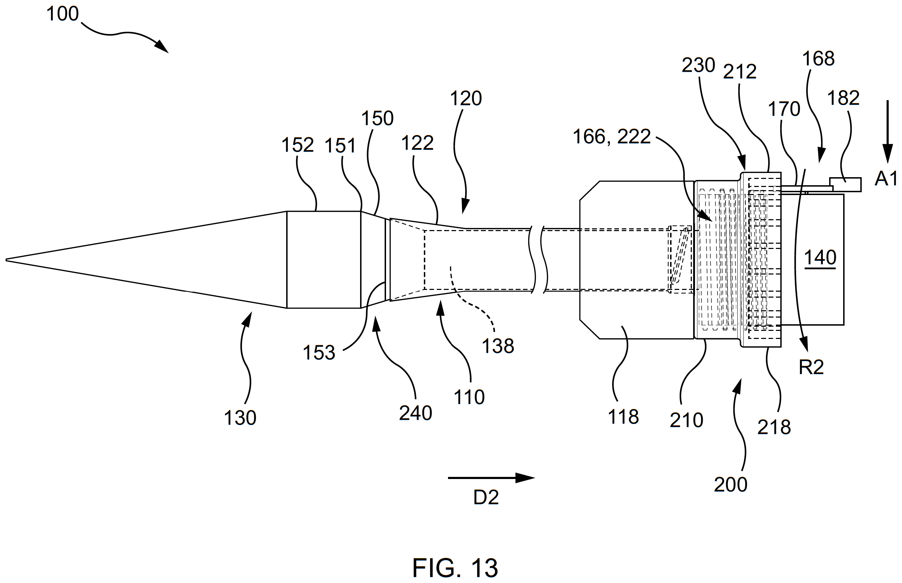

In embodiments herein, the dilator hub 140 includes a lock assembly 168 including an arm or tab 170 and a biasing element 172. The lock assembly 168 is disposed adjacent to an outer surface 167 of a second portion 169 the dilator hub 140 proximal of and adjacent to the distal portion 164. The tab 170 includes a first or distal end 174, a second or proximal end 176, an outer surface 178, and an inner surface 180. The lock assembly 168 includes a first state and a second state. In embodiments, the tab 170 is configured to selectively engage a slot of the coupling ring 200, as described below. When the lock assembly 168 is in the first or locked state, the inner surface 180 of the tab 170 is spaced radially outward from the outer surface 167 of the second portion 169 of the dilator hub 140 at a first radial distance RD1, as shown in FIG. 5. When the lock assembly 168 is in a second or unlocked state, the inner surface 180 of the tab 170 is spaced radially outward from the outer surface 167 of the second portion 169 of the dilator hub 140 at a second radial distance, shown in FIG. 13, wherein the inner surface 180 of the tab 170 generally abuts the outer surface 167 of the second portion 169 of the dilator hub 140. Accordingly, in embodiments herein, the first radial distance RD1 is larger than the second radial distance. The second radial distance is not shown because if the inner surface 180 of the tab 170 abuts the outer surface 167 of the second portion 169, the second radial distance is zero. However, this is not meant to be limiting, and the second radial distance may be greater than zero provided that the outer surface 178 of the tab 170 clears *(i.e.,* is radially inward of) walls of the coupling ring 200, as described below.

In embodiments, the tab 170 includes an actuator 182 disposed at the second end 176 of the tab 170, as shown in FIGS. 5-6. The actuator 182 is configured for user manipulation of the tab 170 such that user may toggle or transition the lock assembly 168 between the first state and the second state. In other words, the actuator 182 may be actuated to transition the tab 170 between the first radial distance RD1 from the outer surface 167 of the second portion 169 and the second radial distance from the outer surface 167 of the second portion 169 of the dilator hub 140. The actuator 182 may be of any suitable shape for the purposes described herein. The actuator 182 may be formed for example, and not by way of limitation, as an integral section of the tab 170, or as a separate unit coupled to tab 170 by any suitable method such as, but not limited to adhesives, welding, or other methods as appropriate.

In embodiments, the biasing element 172 is configured to bias or urge the lock assembly 168 towards the first state. In other words, the biasing element 172 may be configured to return the tab 170 towards the first radial distance RD1 from the outer surface 167 of the second portion 169 of the dilator hub 140 when the actuator 182 is not actuated by the user. In an embodiment, the biasing element 172 may be a spring or coil including a first end 184 coupled to the tab 170 and a second end 186 coupled to the housing 156 of the dilator hub 140, as shown in FIG. 5. The first end 184 may be coupled to the tab 170 and the second end 186 may be coupled to the housing 156 of the dilator hub 140 by methods including, but not limited to adhesives, welding, or other methods as appropriate. While the biasing element 172 is described herein with the first end 184 coupled to the tab 170, in embodiments the first end 184 may be coupled to the actuator 182. To transition the lock assembly 168 from the first state to the second state, a user presses the actuator 182 radially inward with sufficient force to overcome the radially outward force of the biasing element 172. When the actuator 182 is released, the force of the biasing element 172 returns the lock assembly 168 to the first state.

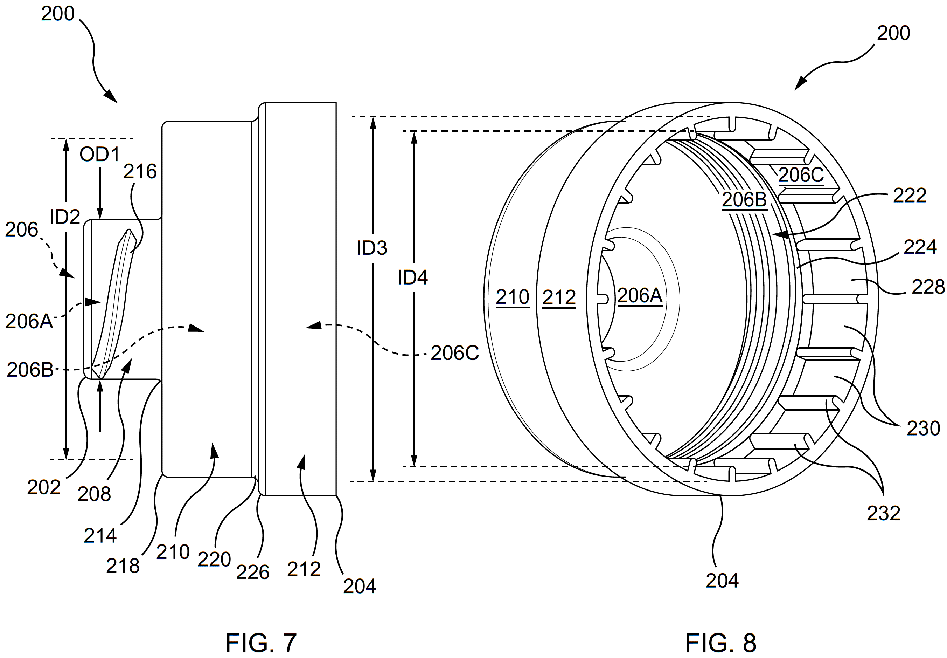

FIGS. 7-8 illustrate an embodiment of the coupling ring 200 according to embodiments herein. The coupling ring 200 includes a first or distal end 202, a second or proximal end 204, and a central lumen 206 extending from the first end 202 to the second end 204. In embodiments, the coupling ring 200 includes a first or distal section 208, a second or middle section 210, and a third or proximal section 212. The first section 208 of the coupling ring 200 extends from the first end 202 to a second or proximal end 214. The first section 208 defines a first portion 206A of the central lumen 206 and is configured to receive a portion of the dilator shaft 138 of the dilator 130. The first section 208 is sized to be received within a proximal portion of the introducer hub 118 to releasably couple the coupling ring 200 to the introducer hub 118, as described below. Accordingly, a first outer diameter OD1 of the first section 208 of the coupling ring 200 is smaller than the first inner diameter ID1 of the central lumen 116 of the introducer 110 at the second end 114 of the introducer hub 118. In the embodiment of FIG. 7, the first section 208 of the coupling ring 200 includes a pair of quarter-turn second coupling threads 216 on an outer surface thereof. The second coupling threads 216 are configured to engage the corresponding first coupling thread 124 of the introducer hub 118 to releasably couple the coupling ring 200 to the introducer hub 118 of the introducer 110. The second coupling threads 216 may be formed for example, and not by way of limitation, as an integral section of the first section 208 of the coupling ring 200, or as separate units coupled to the first section 208 by any suitable method such as, but not limited to adhesives, welding, or other methods as appropriate. While the second coupling threads 216 of the first section 208 are shown in FIGS. 4 and 7 with a specific gender, thread form, thread angle, lead, pitch, and start, this is not meant to limit the design and other configurations and combinations may be utilized. It is understood that the second coupling threads 216 will correspond to the first coupling thread 124 such that the first coupling thread 124 and second coupling threads 216 may be threadably engaged.

The second section 210 of the coupling ring 200 extends from a first end 218 adjacent to the second end 214 of the first section 208 to a second or proximal end 220 of the second section 210. The second section 210 defines a second portion 206B of the central lumen 206 and is configured to receive the distal portion 164 of the dilator hub 140, as shown in FIG. 4. Accordingly, a second inner diameter ID2 of the second portion 206A of the central lumen 206 is greater than a second outer diameter OD2 of the distal portion 164 of the dilator hub 140. The second section 210 includes a second adjusting thread 222 disposed on an inner surface 224 thereof, as shown in FIG. 8. The second adjusting thread 222 is configured to engage the first adjusting thread 162 of the dilator hub 140, as shown in FIG. 4. The second adjusting thread 222 may be formed for example, and not by way of limitation, as an integral section of the second section 210 of the coupling ring 200, or as a separate unit coupled to the second section 210 by any suitable method such as, but not limited to adhesives, welding, or other methods as appropriate. While the second adjusting thread 222 is shown in FIGS. 7-8 with a specific gender, thread form, thread angle, lead, pitch, and start, this is not meant to limit the design and other configurations and combinations may be utilized. It is understood that the second adjusting thread 222 will correspond to the first adjusting thread 162 such that the first and second adjusting threads 162, 222 are configured to be threadably engaged.

In embodiments, as shown in FIGS. 7-8, the third section 212 of the coupling ring 200 includes a first or distal end 226 disposed adjacent to the second end 220 of the second section 210, and a second end of the third section 212 that corresponds to the second end 204 of the coupling ring 200. The third section 212 defines a third portion 206C of the central lumen 206 of the coupling ring 200. The third portion 206C of the central lumen 206 is configured to receive the middle portion 146 of the dilator hub 140 and a portion of the tab 170 of the lock assembly 168, as shown in FIG. 4. The third portion 206C of the central lumen 206 is defined by an inner surface 228 of the third section 212, as shown in FIG. 8. The third section 212 includes a plurality of slots 230 arranged circumferentially around the inner surface 228 of the third section 212. The plurality of slots 230 are formed by a plurality of spaced-apart walls 232 extending radially inwardly from the inner surface 228. The plurality of slots 230 are defined in the spaces between circumferentially adjacent walls 232. The third section 212 includes a third inner diameter ID3 defined by the inner surface 228 and a fourth inner diameter ID4 defined by an inner surface of the plurality of walls 232. Thus, the third inner diameter ID3 also defines the radial diameter of the slots 230. In embodiments herein, the third inner diameter ID3 is larger than the fourth inner diameter ID4 defined by the inner surface of the walls 232. The fourth inner diameter ID4 defined by the inner surface of the plurality of walls 232 is larger than a third outer diameter OD3 of the middle portion 146 of the dilator hub 140 by at least the thickness of the tab 170 of the lock assembly 168 such that the middle portion 146 of the dilator hub 140 may rotate within the third section 212 of the coupling ring 200 when the lock assembly 168 is in the second state (unlocked). Each slot 230 is configured to selectively receive a distal portion of the tab 170 of the lock assembly 168 when the slot 230 is rotationally aligned with the tab 170 and the tab 170 is spaced the first radial distance RD1 from the outer surface 178 of the middle portion 146 of the dilator hub 140, as shown in FIG. 4. When the tab 170 is disposed within one of the slots 230, the tab 170 is spaced from the outer surface 178 the first radial distance RD1, the lock assembly 168 is in the first or locked state and the dilator 130 is rotationally locked to the coupling ring 200, as described below. Stated another way, with the tab 170 engaged within one of the slots 230, the tab 170 prevents rotational movement of the dilator hub 140 relative to the coupling ring 200. When the dilator hub 140 is releasably coupled to and rotationally locked with the coupling ring 200, the assembly is in a first or locked configuration.

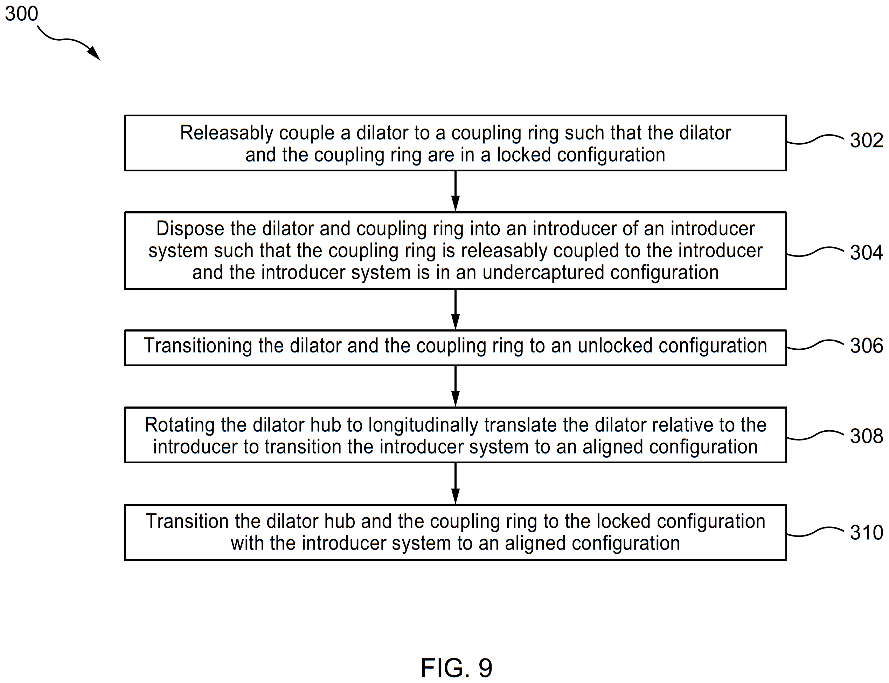

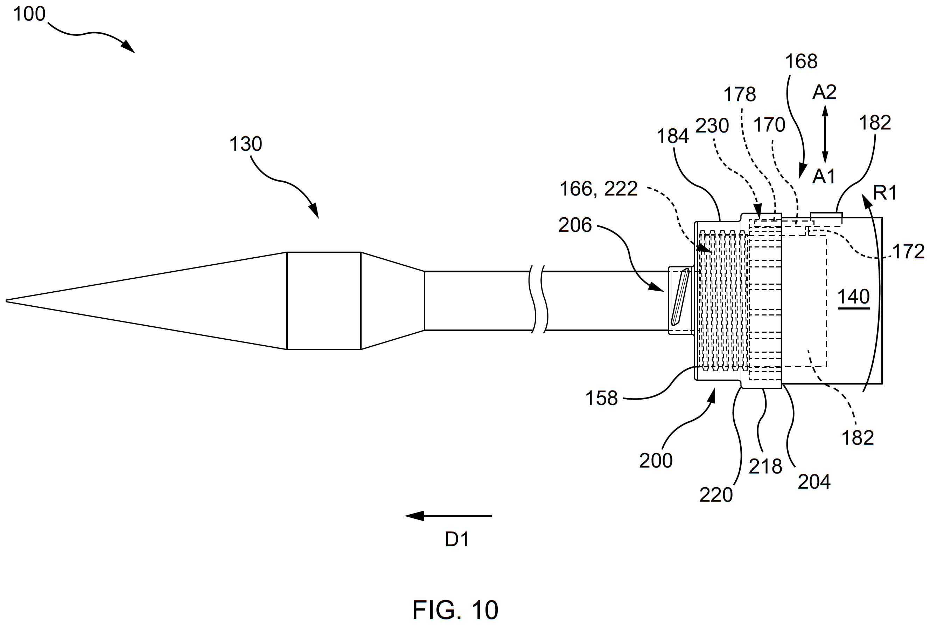

With an understanding of the components of the introducer system 100 above, the interactions of the various components and methods for precisely adjusting the longitudinal or axial position of the distal tip 122 of the introducer 110 relative to the distal portion 148 by rotation of the dilator 130 to optimize the transition interface therebetween are described with respect to FIGS. 9-15. FIG. 9 is a flow chart showing a method 300 of adjusting the longitudinal position of the dilator 130 relative to the introducer 110. FIGS. 10-15 shows steps of the method 300 to adjust the dilator 130 relative to the introducer 110. One skilled in the art will realize that FIGS. 9-15 illustrate one example of a method of adjusting the transition interface between the dilator and the introducer and that certain steps of the method may be removed or combined, and that additional steps of the method may be performed in keeping with scope of the present disclosure.

In a step 302 of the method 300 illustrated in FIG. 9, the dilator 130 and the coupling ring 200 are set to the first or locked configuration. In particular, in the first or locked configuration, the dilator 130 is releasably coupled to the coupling ring 200 with the dilator hub 140 of the dilator 130 in its distal-most position relative to the coupling ring 200 and the locking assembly 168 of the dilator hub 140 is in the first state such that the dilator 130 is prevented from rotational movement relative to the coupling ring 200, as shown in FIG. 10. In greater detail, with the lock assembly 168 of the dilator hub 140 in the first state, the distal tapered tip 154 of the dilator 130 is inserted into the central lumen 206 at the second end 204 of the coupling ring 200, and the dilator 130 is moved in a first or distal direction indicated by an arrow D1 in FIG. 10 until the first end 158 of the dilator hub 140 abuts the second (proximal) end 220 of the second section 210 of the coupling ring 200. A user actuates or presses the actuator 182 of the lock assembly 168 radially inward in the direction of the arrow A1 in FIG. 10. The tab 170 of the lock assembly 168 moves radially inward such that the outer surface 178 of the tab 170 is radially clear *(i.e.,* radially inward) of the walls 232 of the coupling ring 200 such that the dilator hub 140 is free and may be rotated relative to the coupling ring 200. The dilator hub 140 is rotated in the first direction R1 such that the first adjusting thread 162 of the dilator hub 140 engages the second adjusting thread 222 of the coupling ring 200. In particular, the dilator hub 140 is rotated in the first direction D1 until the dilator hub 140 is in its distal-most position relative to the coupling ring 200. Thus, the second threads 222 of the coupling ring 200 are fully engaged with the first adjusting threads 162 of the dilator hub 140. With the dilator hub 140 in its distal-most position relative to the coupling ring 200, the actuator 182 is released such that the biasing element 172 applies a radially outward force to the tab 170 such that the tab 170 moves radially outward in the direction of the arrow A2 in FIG. 10 and into one of the slots 230 of the third section 212 of the coupling ring 200. With the tab 170 disposed within one of the slots 230 of the coupling ring 200, the dilator 130 and the coupling ring 200 coupled thereto are in the first or locked configuration.

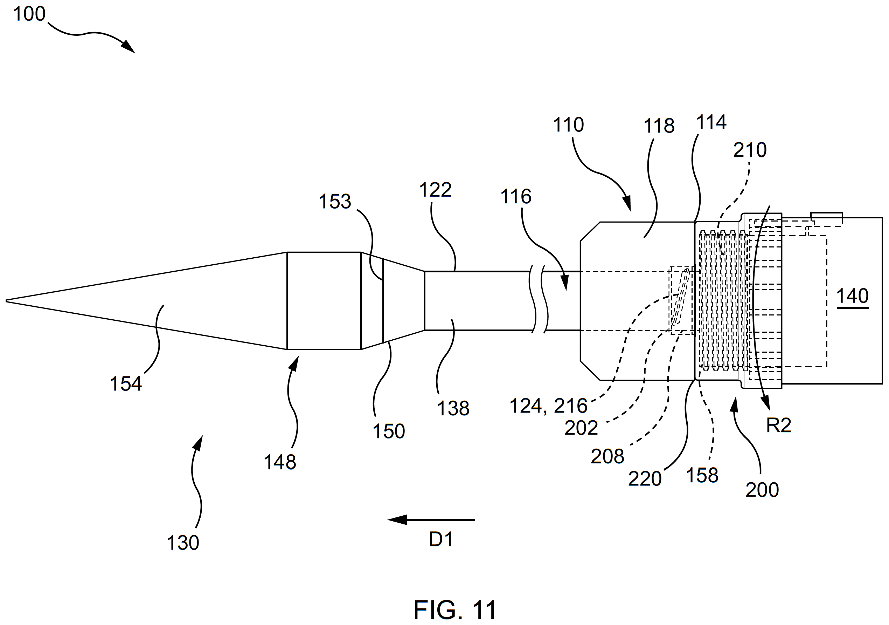

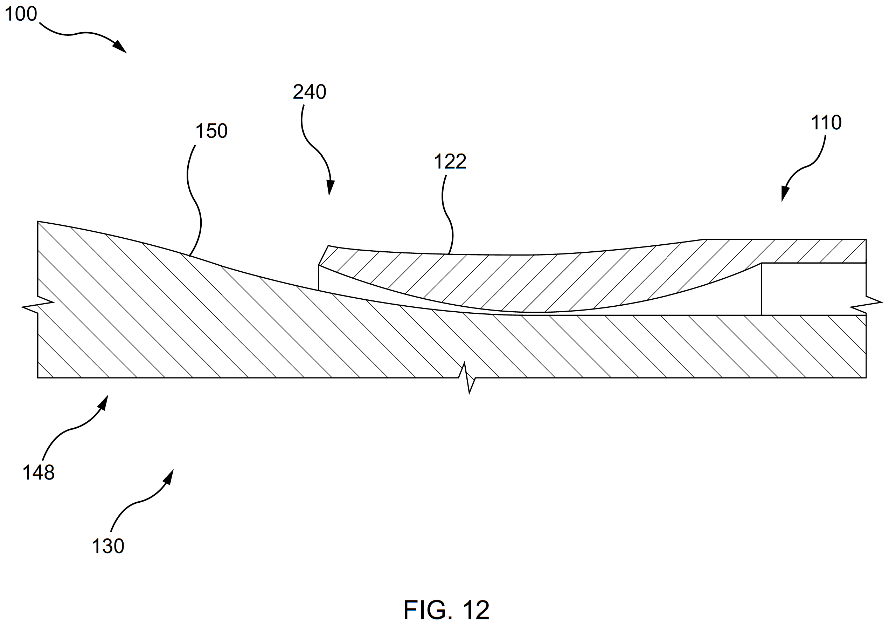

In a step 304 of the method 300, the dilator 130 is inserted through the central lumen 116 of the introducer 110 of the introducer system 100 such that the coupling ring 200 is releasably coupled to the introducer hub 118 and the introducer system 100 is in an undercaptured configuration, as shown in FIGS. 11 and 12. In particular, the distal tapered tip 154 of the dilator 130 is inserted into the central lumen 116 at the second end 114 of the introducer 110, and the dilator 130 is moved in the first or distal direction indicated by the arrow D1 in FIG. 11 until the first end 202 of the first section 208 of the coupling ring 200 abuts the second end 220 of the introducer hub 118. The coupling ring 200 is rotated in a second direction R2 opposite the first direction R1 such that the second coupling threads 216 of the coupling ring 200 engage the first coupling threads 124 of the introducer hub 118. The coupling ring 200 is rotated in the second direction R2 until the first coupling threads 124 of the introducer hub 118 are fully engaged with the second coupling threads 216 of the coupling ring 200 such that the coupling ring 200 is releasably coupled to the introducer hub 118 and in its distal-most position relative to the introducer hub 118. With the coupling ring 200 in its distal-most portion relative to the introducer hub 118, the distal portion 148 of the dilator 130 is disposed distal of the distal tip 122 of the introducer 110 in the undercaptured configuration. In the undercaptured configuration, the distal tip 122 of the introducer 110 is disposed proximally of an optimal position relative to the dilator shaft 138. In the particular embodiment shown, in the undercaptured configuration, the distal tip 122 of the introducer 110 is disposed adjacent a proximal end of the distal transition portion 150 of the dilator shaft 138. In the undercaptured configuration, the first (distal) end 158 of the dilator hub 140 is disposed adjacent to and/or abutting the first (distal) end 218 of the second section 210 of the coupling ring 200.

In a step 306 of the method 300, the dilator 130 and the coupling ring 200 are transitioned to an unlocked configuration. In particular, the lock assembly 168 is transitioned from the first state to the second stated by actuating, pressing, or pushing the actuator 182 radially inward in the direction of the arrow A1, as shown in FIG. 13. Actuation of the actuator 182 radially inward moves the tab 170 radially inward and the tab 170 exits or disengages from one of the slots 230 of the third section 212 of the coupling ring 200. With the tab 170 of the lock assembly 168 disengaged from one of the slots 230 of the coupling ring 200, the dilator 130 and the coupling ring 200 are in the unlocked configuration and the dilator 130 is free to rotate relative to the coupling ring 200.

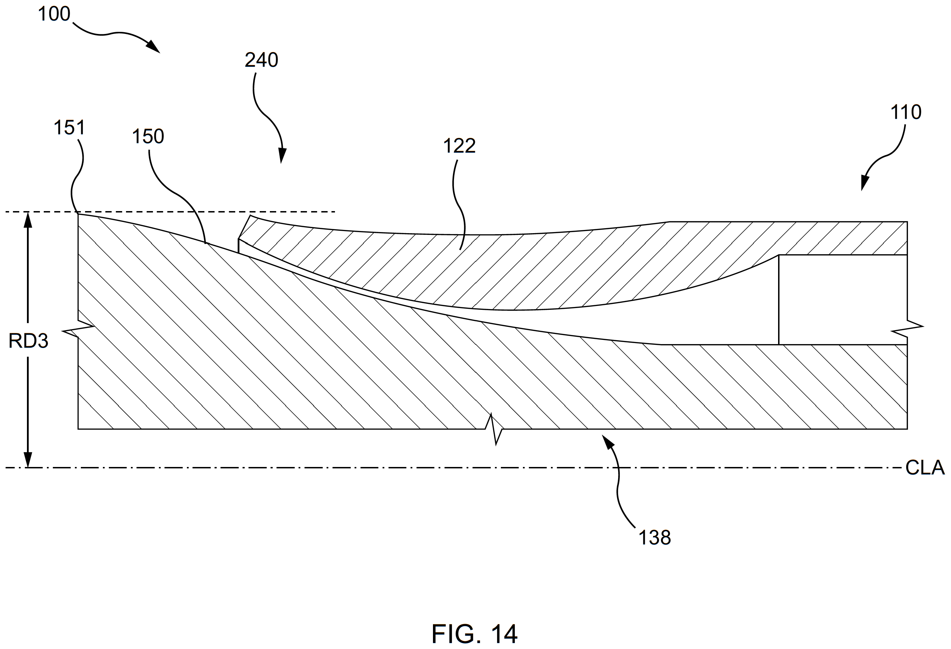

In a step 308 of the method 300, the dilator 130 is rotated to finely and precisely adjust the position of the dilator 130 relative to the introducer 110 such that the distal tip 122 of the introducer sheath 120 is disposed in an aligned configuration relative to the dilator shaft 138, as shown in FIGS. 13 and 14. With the lock assembly 168 in the second state, *i.e.,* by continuing to press the actuator 182 radially inward, the dilator hub 140 is rotated in the second direction R2 to transition the introducer system 100 from the undercaptured configuration to the aligned configuration. In greater detail, with the coupling ring 200 releasably secured to the introducer hub 118 and the lock assembly 168 in the second state, rotating the dilator hub 140 in the second direction R2 causes the first adjusting thread 162 of the dilator hub 140 to rotate along the second adjusting thread 222 of the coupling ring 200, thereby causing the dilator hub 140, and accordingly the dilator shaft 138 coupled thereto, to translate longitudinally in the second longitudinal direction D2 *(i.e.,* proximally). Thus, rotation of the dilator hub 140 in the second direction R2 moves, translates, pulls, or draws the dilator shaft 138 in the second longitudinal direction D2 (proximally) relative to the introducer 110. The dilator hub 140 is rotated in the second direction R2 until the introducer system 100 is in the aligned configuration shown in FIGS. 13 and 14. In particular, the introducer system 100 is in the aligned position when the distal tip 122 of the introducer 110 is just proximal of a distal end 151 of the transition portion 150 of the dilator shaft 138. The aligned position can also be considered as when an outer surface of the cylindrical portion 152 (or the distal end 151 of the transition portion 150) and an outer surface of the distal tip 122 present a smooth transition interface 240. In other words, a smooth transition interface 240 is present when the outer surface of the distal tip 122 and the outer surface of the cylindrical portion 152/distal end 151 of the transition portion 150 are substantially radially aligned *(i.e.,* disposed at a substantially similar radial distance RD3 from a central longitudinal axis CLA of the dilator shaft 138, as shown in FIG. 14). Such a smooth transition interface 240 minimizes gaps or bumps in the transition interface 240, minimizing insertion forces and minimizing potential trauma to the patient during insertion and tracking through the vasculature of the patient.

In a step 310 of the method 300, with the introducer system 100 in the aligned configuration, the lock assembly 168 is transitioned to the first (locked) state. In particular, the user releases the actuator 182 of the lock assembly 168. Upon release of the actuator 182, the biasing element 172 applies a radially outward force in the direction of the arrow A2 such that the tab 170 moves radially outward. As the tab 170 moves radially outward, a portion of the tab 170 engages one of the slots 230 of the third section 212 of the coupling ring 200. When the tab 170 is engaged in one of the slots 230 between the adjacent walls 232, the dilator 130/coupling ring 200 assembly is in the locked configuration such that the dilator hub 140 may not be rotated relative to the coupling ring 200, thereby preventing the longitudinal movement or translation of the dilator 130 relative to the introducer 110.

While embodiments of coupling rings have been described herein as being disposed on the dilator of the introducer system, this is not meant to be limiting, and in other embodiments the coupling ring may be disposed on the introducer of the introducer system.

Embodiments herein have been described with a coupling ring enabling rotary movement to cause longitudinal translation for fine adjustment of the dilator relative to the introducer. However, this too is not meant to be limiting. In other embodiments, a linear adjustment or any other suitable mechanism configured to move the introducer relative to the dilator adjust the transition interface between the introducer and the dilator to an aligned configuration prior to introduction into the vasculature of a patient.

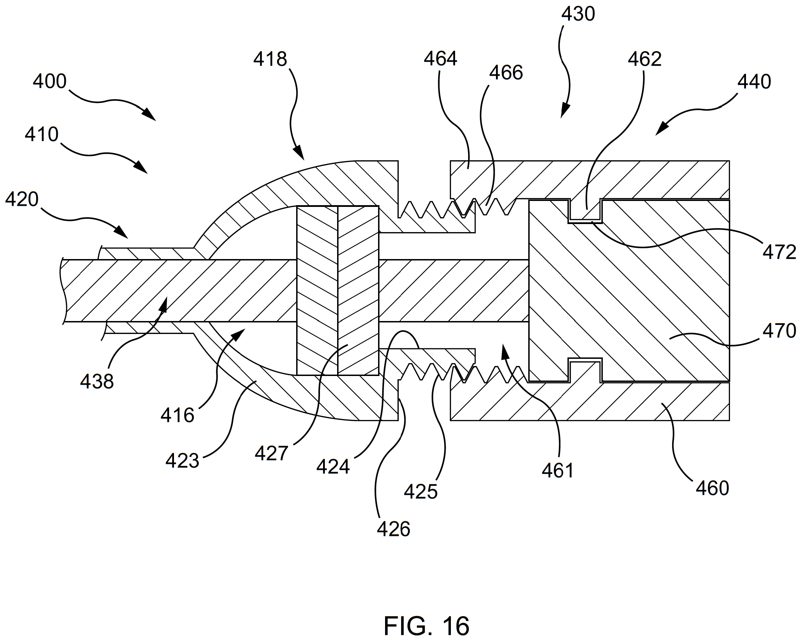

FIGS. 16-19 show an introducer system 400 according to embodiments herein. The introducer system 400 includes an introducer 410 and a dilator 430. The introducer system 400 is configured to provide a stable central vascular access point and to stretch or expand the vasculature to permit insertion and manipulation of catheters, guidewires, or other medical instruments requiring vascular access. The introducer 410 and the dilator 430 are configured to enable adjustment of the interface between the introducer 410 and the dilator 430 of the introducer system 400 to enable accurate positioning of the distal end of an introducer sheath of the introducer 410 relative to a dilator shaft of the dilator 430, as described below.

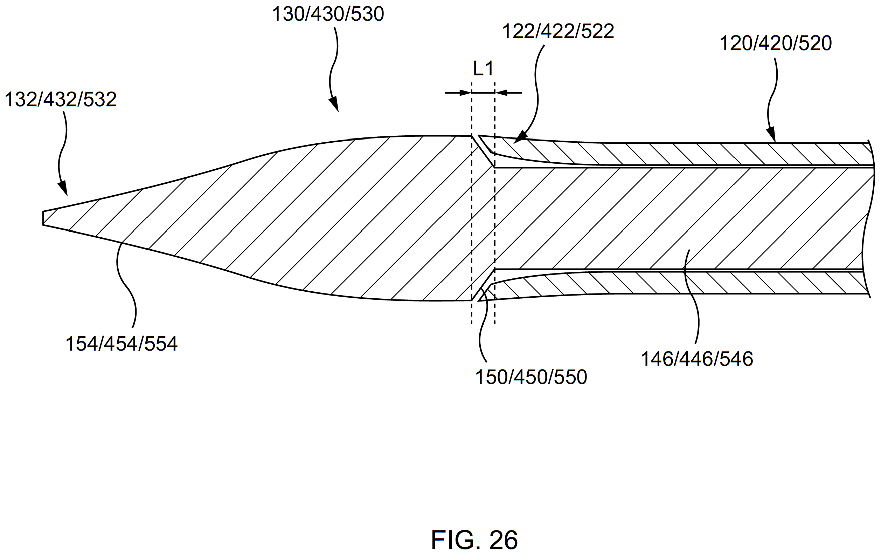

The introducer 410 includes an introducer hub 418, an introducer sheath 420, and a distal tip 422 (see FIG. 26). The distal tip 422 is coupled to and covers a distal end of the introducer sheath 420. However, in other embodiments, the distal tip 422 may be the distal end of the introducer sheath 420. A proximal end of the sheath 420 is coupled to a distal end of the introducer hub 418. Except as described herein, the introducer 410 (including the introducer hub 418, the introducer sheath 420, and the distal tip 422) may be as described in International Patent Publication No. WO 2023/147422 to Medtronic, Inc., and/or International Patent Publication No. WO 2025/003844 to Medtronic, Inc., each of which is incorporated by reference herein in its entirety. As described above, the introducer sheath 420 may be of an elastic or expanding sheath as described, for example, in U.S. Published Application Publication No. 2014/0236122, and/or International Patent Publication No. WO 2025/003844 to Medtronic, Inc., each of which is incorporated by reference herein in its entirety. Further, the introducer 410 may include additional components not shown in FIGS. 16-19, as described in the publications listed above and incorporated by reference herein. The introducer sheath 420 generally includes a flexible, radially expandable shaft extending from the introducer hub 418 to the distal tip 422. The introducer 410 includes a central lumen 416 defined in part by the introducer hub 418 and in part by the introducer sheath 420 and the sheath tip 422.

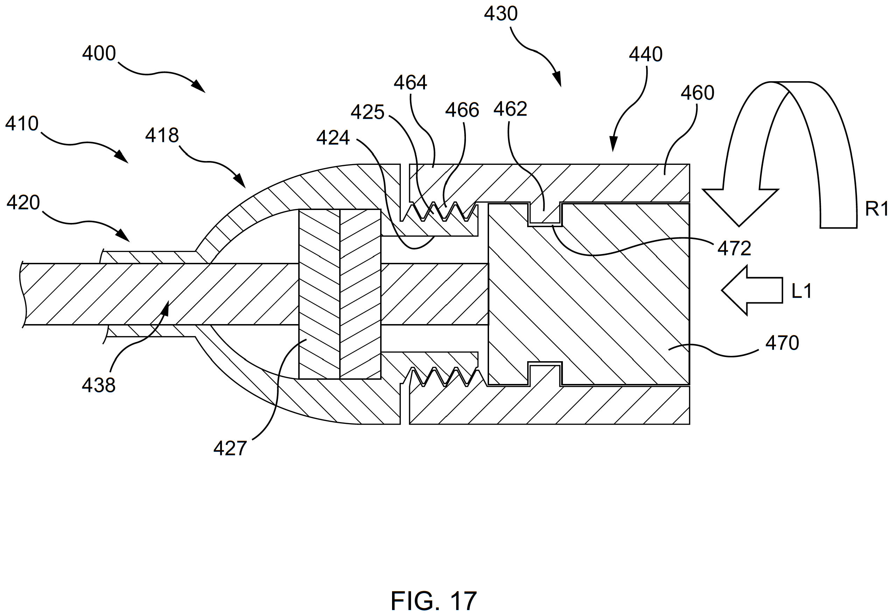

The introducer hub 418 includes a body 423 and a proximal extension 424 extending proximally from the body 423, as shown in FIGS. 16-19. In the embodiment shown, the proximal extension 424 is disposed radially inward of an outer surface of the body 423, thereby forming a shoulder 426 between the body 423 and the proximal extension 424. The proximal extension 424 may be generally cylindrical and defines part of the central lumen 416 therewithin. An outer surface of the proximal extension 424 includes threads 425 configured to interact with corresponding threads of a dilator hub 440 of the dilator 430, as described in more detail below. The introducer hub 418 may include other features as described above and in the publications listed above, such as, but not limited to, seals 427, shown schematically in FIGS. 16 and 17.

FIGS. 16-19 also show the dilator 430. The dilator 430 may be configured to be used with an expandable introducer, such as, but not limited to the introducer 410 described herein. However, this is not meant to be limiting, and the dilator 430 may be used with other expandable introducers and other expandable sheaths. Those skilled in the art will recognize that features may be added and/or removed from the dilator 430.

As shown in FIGS. 16-19, the dilator 430 includes a dilator shaft 438 coupled to and extending distally from the dilator hub 440. The dilator shaft 438 includes a first or distal end that defines a first or distal end 432 of the dilator 430 and a second or proximal end coupled to the dilator hub 440. The dilator shaft 438 defines a distal portion of a central lumen of the dilator 430. The dilator shaft 438 may further include a proximal portion, a middle portion, and a distal portion, as described above and incorporated into the embodiment of FIGS. 16-19. A distal transition portion 450 of the dilator shaft 438, similar to the distal transition portion 150 described above, will be described below with respect to FIG. 26.

The dilator hub 440 includes an outer body or housing 460 and an inner body or housing 470 disposed within the outer body 460. The dilator shaft 438 is coupled to and extends distally from the inner body 470. The inner body 470 defines a proximal portion of the lumen of the dilator 430. An outer surface of the inner body 470 includes a notch or recess 472 extending circumferentially around the inner body 470 for receiving a portion of the outer body 460, as described below. Longitudinal movement/translation of the inner body 470 correspondingly longitudinally moves/translates the dilator shaft 438, as described below.

The outer body 460 of the dilator hub 440 may be generally cylindrical and may include a central passage 461 in which the inner body 470 is disposed. An inner surface of the outer body 460 includes a projection or tab 462 extending radially inward to couple the outer body 460 to the inner body 470, while allowing rotational movement between the outer body 460 and the inner body 470. In the embodiment shown, the tab 462 of the outer body 460 extends into a recess 472 in an outer surface of the inner body 470, as shown in FIGS. 16 and 17. As FIGS. 16 and 17 are cross-sectional views, the tab 462 and recess 472 are shown on the upper and lower portions of FIGS. 16 and 17. However, the tab 462 and the recess 472 in this embodiment both extend circumferentially around the inner surface of the outer body 460 and the outer surface of the inner body 470, respectively, thereby enabling rotation of the outer body 460 relative to the inner body 470 while transferring longitudinal movement of the outer body 460 to the inner body 470.

An inner surface of a distal portion 464 of the outer body 460 defines an internally threaded region including threads 466 configured to mate with the threads 425 formed on the outer surface of the proximal extension 424 of the introducer hub 418. The threaded engagement of the threads 466 of the outer body 460 of the dilator hub 440 and the threads 425 of the introducer hub 418 convert rotation of the outer body 460 to translation of the outer body 460, and the inner body 470 coupled thereto, relative to the introducer hub 418. In particular, as shown in FIG. 17 as compared to FIG. 16, rotation of the outer body 460 in a first rotational direction R1 when the threads 425/466 are engaged causes the outer body 460 to move longitudinally towards the introducer hub 418. The engagement between the tab 462 of the outer body 460 and the recess 472 of the inner body 470 causes the longitudinal movement of the outer body 460 to correspondingly longitudinally move the inner body 470, which causes the dilator shaft 438 to longitudinally move via its coupling to the inner body 470. Accordingly, as described, the dilator shaft 438 can be positioned in a desired position relative to the introducer sheath 420 via rotation of the outer body 460. Converting rotational movement of the outer body 460 to translation of the outer body 460, inner body 470, and dilator shaft 438 relative to the introducer hub 418 and the introducer sheath 420 enables precise positioning of the dilator shaft 438 relative to the distal end/sheath tip 422 of the introducer sheath 420.

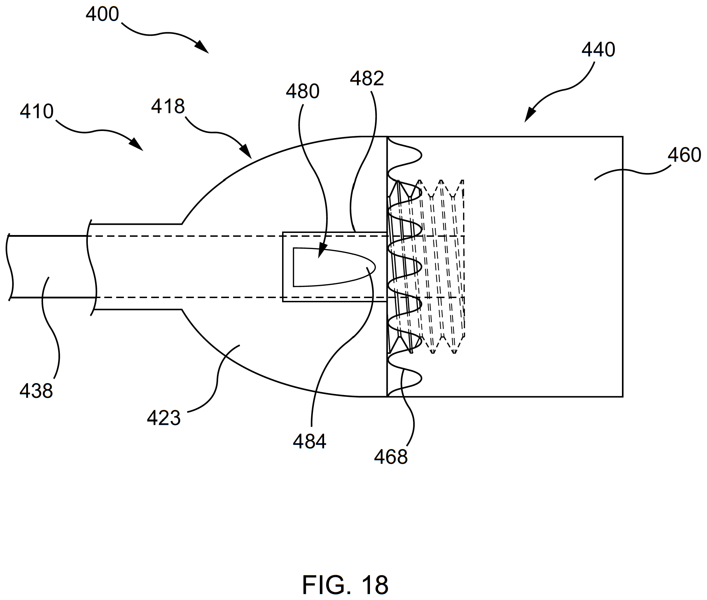

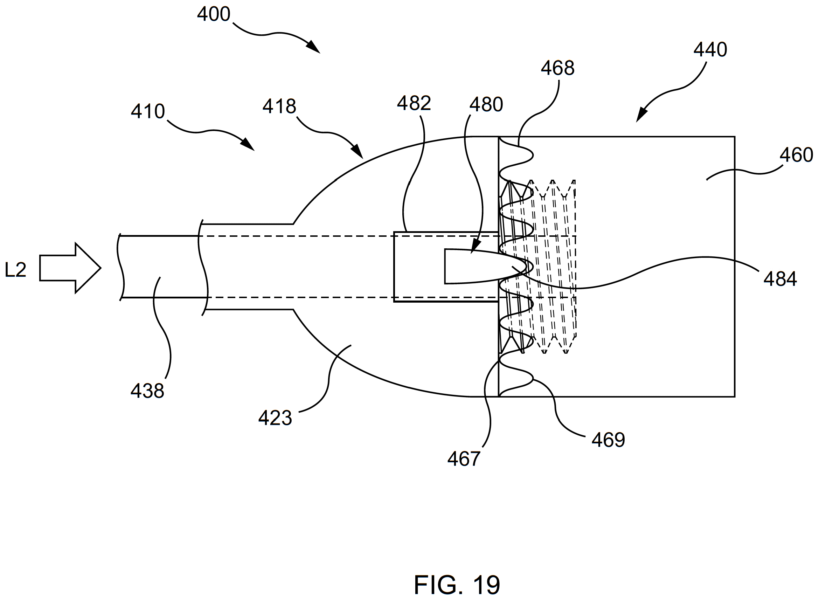

As discussed in the embodiments above, when the desired relative position between the dilator shaft 438 and the sheath tip 422 is achieved, it is desirable to lock or hold that position such that the position does not change when utilizing the introducer system 400. Accordingly, in the embodiment of FIGS. 16-19, and as shown in FIGS. 18 and 19, the introducer system 400 includes a lock configured to maintain a desired relative position of the dilator 430 with respect to the introducer 410. In the embodiment of FIGS. 16-19, the lock is formed by ridges 468 at the distal portion 464 of the outer body 460 of the dilator hub 440 and a slider 480 coupled to the body 423 of the introducer hub 418. In greater detail, the ridges 468 are in the form of a wave or wavy distal end of the outer body 460 forming peaks 467 extending distally and valleys 469 extending proximally. The ridges 468 extend around the circumference of the outer body 460. In the embodiment shown, the slider 480 is disposed within a recess 482 in the outer surface of the body 423 of the introducer hub 418. The slider 480 is configured to move longitudinally between a distal position shown in FIG. 18, in which the introducer hub 418 is not rotationally locked relative to the dilator hub 440, and a proximal position, shown in FIG. 19, in which the introducer hub 418 and the dilator hub 440 are rotationally locked relative to each other. The slider 480 includes a proximal end 484 that is shaped to fit within one of the ridges 468 in the distal portion of the outer body 460 of the dilator hub 440. In particular, the shaped proximal end 484 of the slider 480 is shaped to fit between two adjacent peaks 467 of the ridges 468 of the outer body 460 of the dilator hub 440. The slider 480 may be configured to move longitudinally between the distal position and the proximal position by the manner in which the slider 480 is coupled to the introducer hub 418. For example, and not by way of limitation, the body 423 may include a longitudinal slot (not shown) in the recess 482. Correspondingly, the slider 480 may include a post or tab extending into the longitudinal slot to couple the slider to the body 423 and to enable longitudinal movement of the slider 480 along the slot. The slot may include pinched regions at the proximal and distal ends thereof to retain the slider 480 in place until a user slides the slider 480. Other coupling mechanisms may also be used to enable the slider to slide relative to the body 423 of the introducer hub 418.

Thus, in operation, the slider 480 in the distal position shown FIG. 18 such that the outer body 460 of the dilator hub 440 is free to rotate relative to the introducer hub 418, the threads 425/466 of the dilator hub 418 and the outer body 460, respectively, are brought into engagement. The outer body 460 is rotated relative to the dilator hub 418 and the inner body 470, thereby causing the outer body 460, the inner body 470, and the dilator shaft 438 to translate distally relative to the introducer 410. When the dilator shaft 438 reaches the desired position relative to the introducer sheath 420, that is, when the sheath tip 422 is located at the distal transition portion 450 of the dilator shaft 438, rotation of the outer body 460 is stopped. The slider 480 is then translated proximally such that the shaped proximal end 484 of the slider 480 engages the ridge of the plurality of ridges 468 that is aligned shaped proximal end 484 of the slider 480, thereby preventing relative rotational movement of the outer body 460 relative to the introducer hub 418. Because the threads 425/466 are also engaged, relative longitudinal movement between the dilator hub 440 and the introducer hub 418 without relative rotation is also prevented, thereby locking the relative position of dilator 430, and hence the dilator shaft 438, with respect to the introducer 410, and hence the distal tip 422 of the introducer sheath 420.

It would be understood by those skilled in the art that the embodiment shown in FIGS. 16-19 may be modified without departing from the spirit of the invention. For example, and not by way of limitation, the lock may be reversed such that the slider is disposed on the outer body of the dilator hub, the ridges are disposed on the proximal end of the introducer hub, and the slide is moved distally to engage the ridges to lock the dilator hub relative to the introducer hub.

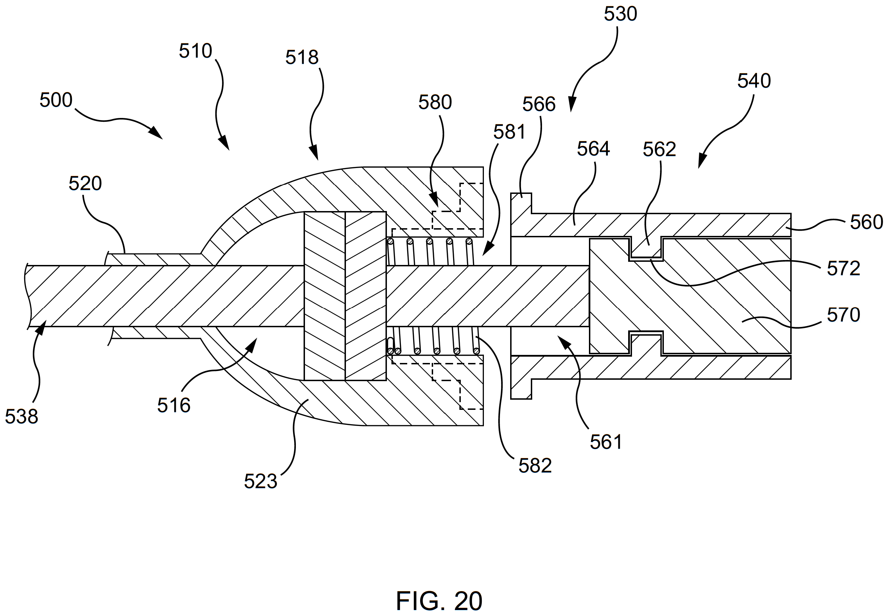

FIGS. 20-25E show an introducer system 500 according to embodiments herein. The introducer system 500 includes an introducer 510 and a dilator 530. The introducer system 500 is configured to provide a stable central vascular access point and to stretch or expand the vasculature to permit insertion and manipulation of catheters, guidewires, or other medical instruments requiring vascular access. The introducer 510 and the dilator 530 are configured to enable adjustment of the interface between the introducer 510 and the dilator 530 of the introducer system 500 to enable accurate positioning of the distal end of an introducer sheath of the introducer 510 relative to a dilator shaft of the dilator 530, as described below.

Similar to the embodiments above, the introducer 510 includes an introducer hub 518, an introducer sheath 520, and a distal tip 522 (see FIG. 26). The distal tip 522 is coupled to and covers a distal end of the introducer sheath 520. However, in other embodiments, the distal tip 522 may be the distal end of the introducer sheath 520. A proximal end of the sheath 520 is coupled to a distal end of the introducer hub 518. Except as described herein, the introducer 510 (including the introducer hub 518, the introducer sheath 520, and the distal tip 522) may be as described in International Patent Publication No. WO 2023/147422 to Medtronic, Inc., and/or International Patent Publication No. WO 2025/003844 to Medtronic, Inc., each of which is incorporated by reference herein in its entirety. As described above, the introducer sheath 520 may be of an elastic or expanding sheath as described, for example, in U.S. Published Application Publication No. 2014/0236122, and/or International Patent Publication No. WO 2025/003844 to Medtronic, Inc., each of which is incorporated by reference herein in its entirety. Further, the introducer 510 may include additional components not shown in FIGS. 20-25E, as described in the publications listed above and incorporated by reference herein. The introducer sheath 520 generally includes a flexible, radially expandable shaft extending from the introducer hub 518 to the distal tip 522. The introducer 510 includes a central lumen 516 defined in part by the introducer hub 518 and in part by the introducer sheath 520 and the sheath tip 522.

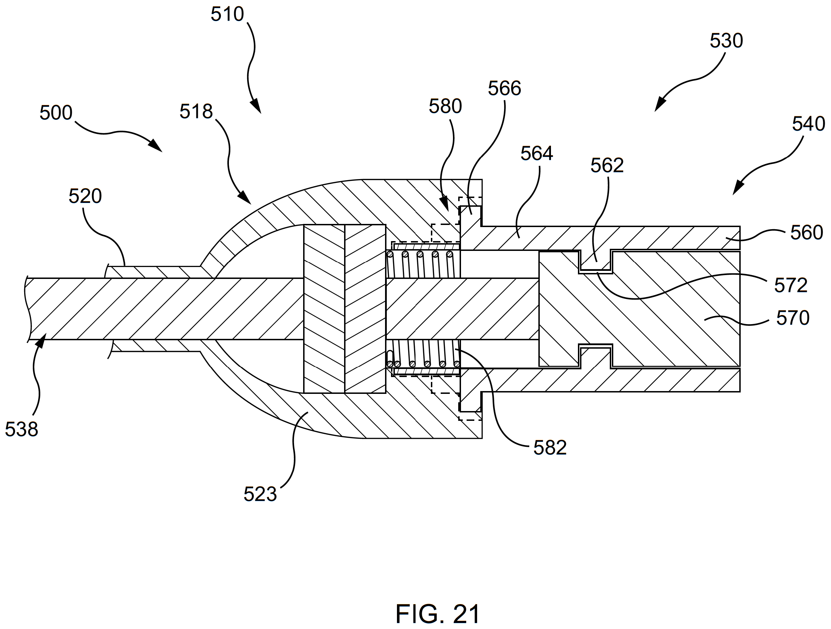

The introducer hub 518 includes a body 523 defining a portion of the central lumen 516 and configured to receive the dilator 530, as described below. A proximal portion of the body 523 includes a circumferential channel 580 configured to receive and retain a portion of a dilator hub 540 of the dilator 530, as described in more detail below. A spring 582 is disposed in the channel 580 and is configured to apply a force on the dilator hub 540 to maintain a position of the dilator hub 540 relative to the introducer hub 518, as described in more detail below. The introducer hub 518 may include other features as described above and in the publications listed above, such as, but not limited to, seals 527, shown schematically in FIGS. 20 and 21.

FIGS. 20-25E also show the dilator 530. The dilator 530 may be configured to be used with an expandable introducer, such as, but not limited to the introducer 510 described herein. However, this is not meant to be limiting, and the dilator 530 may be used with other expandable introducers and other expandable sheaths. Those skilled in the art will recognize that features may be added and/or removed from the dilator 530.

As shown in FIGS. 20 and 21, the dilator 530 includes a dilator shaft 538 coupled to and extending distally from the dilator hub 540. The dilator shaft 538 includes a first or distal end that defines a first or distal end 532 of the dilator 530 and a second or proximal end coupled to the dilator hub 540. The dilator shaft 538 defines a distal portion of a central lumen of the dilator 530. The dilator shaft 538 may further include a proximal portion, a middle portion, and a distal portion, as described above and incorporated into the embodiment of FIGS. 20-25E. A distal transition portion 550 of the dilator shaft 538, similar to the distal transition portion 150 described above, will be described below with respect to FIG. 26.

The dilator hub 540 includes an outer body or housing 560 and an inner body or housing 570 disposed within the outer body 560. The dilator shaft 538 is coupled to and extends distally from the inner body 570. The inner body 570 defines a proximal portion of the lumen of the dilator 530. An outer surface of the inner body 570 includes a notch or recess 572 extending circumferentially around the inner body 570 for receiving a portion of the outer body 560, as described below. Longitudinal movement/translation of the inner body 570 correspondingly longitudinally moves/translates the dilator shaft 538, as described below.

The outer body 560 of the dilator hub 540 may be generally cylindrical and include a central passage 561 in which the inner body 570 is disposed. An inner surface of the outer body 560 includes a projection or tab 562 extending radially inwardly to couple the outer body 560 to the inner body 570, while allowing rotational movement between the outer body 560 and the inner body 570. In the embodiment shown, the tab 562 of the outer body 560 extends into the recess 572 in the outer surface of the inner body 570, as shown in FIGS. 20 and 21. As FIGS. 20 and 21 are cross-sectional views, the tab 562 and recess 572 are shown on the upper and lower portions of FIGS. 20 and 21. However, the tab 562 and the recess 572 in this embodiment both extend circumferentially around the inner surface of the outer body 460 and the outer surface of the inner body 570, respectively, thereby enabling rotation of the outer body 560 relative to the inner body 570 while transferring longitudinal movement of the outer body 560 to the inner body 570.

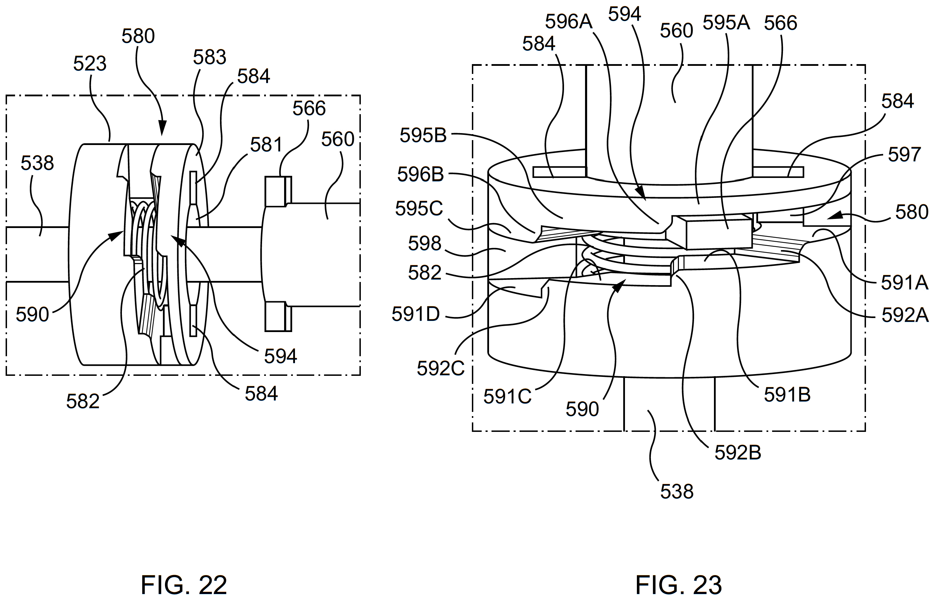

A distal portion 564 of the outer body 560 includes wings or tabs 566 extending radially outwardly. In the embodiment shown, there are two wings 566 disposed approximately 180 degrees apart around the circumference of the outer body 560, as best seen in FIGS. 22 and 23. However, this is not meant to be limiting, and more or fewer wings 566 may be utilized. The wings 566 are configured to interact with the channel 580 in the introducer hub 518 to couple the dilator hub 540 to the introducer hub 518 and to enable precise or fine adjustment of the longitudinal position of the dilator hub 540 relative to the introducer hub 518. Precise adjustment of the longitudinal position of the dilator hub 540 relative to the introducer hub 518 correspondingly adjust the longitudinal position of the dilator shaft 538 relative to the introducer sheath 520, as described below.

FIGS. 22 and 23 show close-up views of the proximal portion of the body 523 of the introducer hub 518 showing details of the channel 580 and its interaction with the dilator 530. While described as part of the proximal portion of the body 523, the channel 580 may instead be part of an insert that is coupled to the body 523. FIGS. 22 and 23 show a single channel 580 for one of the wings 566 of the distal portion 564 of the outer body 560 of the dilator hub 540, but those skilled in the art will recognize that there are two channels 580, one of each of the wings 566. In particular, FIGS. 22 and 23 show one channel 580 facing towards the viewer, but a second channel 580 is on the opposite side (back side in FIGS. 22 and 23) of the body 523.

As can be seen in FIGS. 22 and 23, a proximal facing surface 583 of the body 523 includes a proximal opening 581. The proximal opening 581 provides access to the central lumen 516 of the introducer hub 518. The body 523 of the introducer hub 518 further includes two slots 584 in the proximal facing surface 583. The slots 584 are disposed about 180 degrees apart from each other around the circumference of the proximal opening 581. The slots 584 provide access to the channels 580. The slots 584 are sized and shaped to receive the wings 566 of the outer body 560 of the dilator hub 540 therein, as described below.

Each channel 580 includes a proximal wall 594 and a distal wall 590. The proximal wall 594 and the distal wall 590 are each stepped to enable the wing 566 disposed therein to be held at different longitudinal positions, as described below. Accordingly, the proximal wall 594 includes a series of proximal landings 595A, 595B, 595C and a series of proximal steps 596A, 596B, with each proximal step disposed between an adjacent pair of proximal landings. In particular, the proximal step 596A is disposed between the proximal landing 595A and the proximal landing 595B. The proximal step 596A extends in a distal direction such that the proximal landing 595B is distal of the proximal landing 595A. Similarly, the proximal step 596B is disposed between the proximal landing 595B and the proximal landing 595C. The proximal step 596B extends in a distal direction such that the proximal landing 595C is distal of the proximal landing 595B. Similarly, the distal wall 590 includes a series of distal landings 591A, 591B, 591C, 591D and a series of distal steps 592A, 592B, 592C with each step disposed between an adjacent pair of distal landings. In particular, the distal step 592A is disposed between the distal landing 591A and the distal landing 591B. The distal step 592A extends in a distal direction such that the distal landing 591B is distal of the distal landing 591A. Similarly, the distal step 592B is disposed between the distal landing 591B and the distal landing 591C. The distal step 592B extends in a distal direction such that the distal landing 595C is distal of the distal landing 595B. Similarly, the distal step 592C is disposed between the distal landing 591C and the distal landing 591D. The distal step 592C extends in a distal direction such that the distal landing 591D is distal of the distal landing 591C. A first radial wall 597 extends between the distal landing 591A and the proximal landing 595A to close the channel 580 circumferentially. The first radial wall 597 is disposed adjacent the slot 584 such that the corresponding wing 566 can enter the channel 580 through the slot 584 without the first radial wall 597 blocking the slot 584. A second radial wall 598 extends between the proximal landing 595C and the distal landing 591D to close off an end of the channel 580 circumferentially opposite the corresponding slot 584 to the channel 580. The channel 580 on the opposite side of the shown channel 580 is the same as the channel 580 described herein. Persons skilled in the art would recognize that the quantity, location, and size of the landings and steps shown herein may be varied without departing from the spirit and scope of the invention, provided that the channels enable adjustment of the position of the dilator hub 540 with respect to the introducer hub 518.

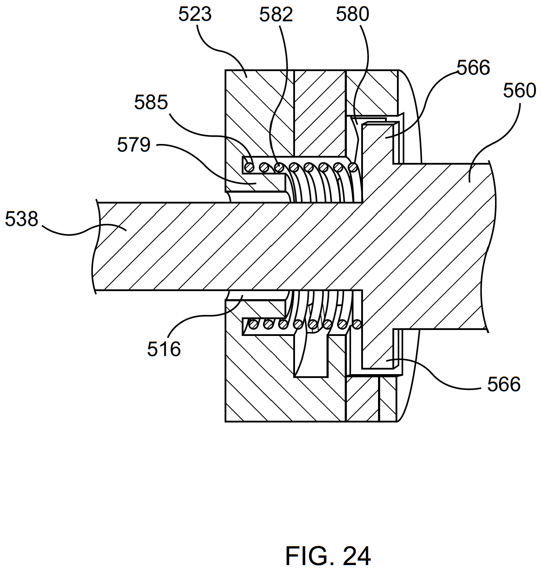

FIG. 24 shows a cross-sectional view of the proximal portion of the body 523 of the introducer hub 518 with the outer body 560 of the dilator hub 540 inserted therein. In particular, the wings 566 of the outer body 560 of the dilator hub 540 are inserted into the channels 580 through the slots 584, but the outer body 560 has not yet been rotated as described below. This cross-sectional view of the proximal portion of the body 523 shows a circumferential channel 585 extending around a cylindrical wall 579 defining the central lumen 516 of the introducer hub 518 and extending longitudinally. The channel 585 intersects with the channel 580. The channel 585 is sized and shaped to hold the spring 582 such that the spring 582 can act on the outer body 560 of the dilator hub 540, as explained below.

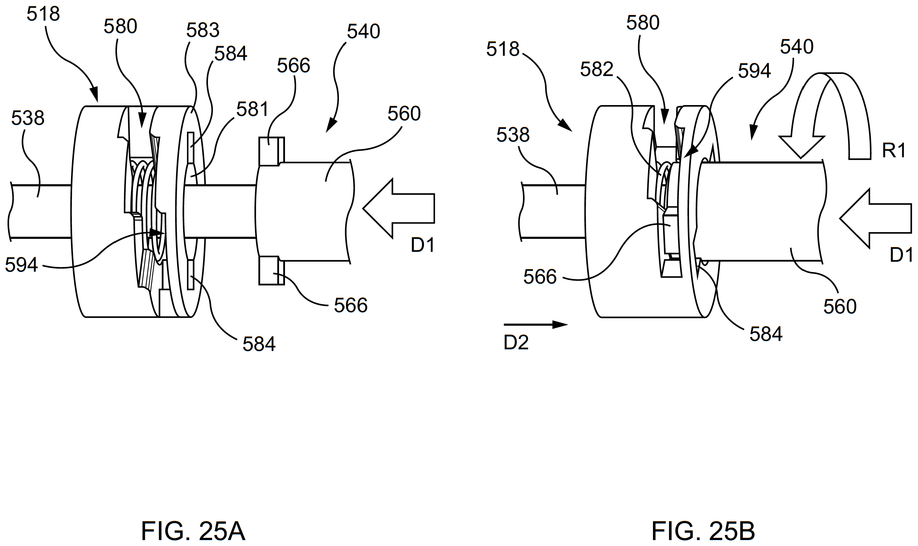

With the parts of the introducer system 500 described, the operation of the introducer 510 and the dilator 530 to couple the introducer 510 and the dilator 530 and to align the distal tip 522 of the introducer sheath 520 relative to the dilator shaft 538 will be described with reference to FIGS. 25A-25E. FIGS. 25A-25E show only a portion of the body 523 of the introducer hub 518 for clarity. Those skilled in the art, relying on FIGS. 20 and 21, knowledge of introducer hubs, and the publications listed above and incorporated by reference herein, will recognize that FIGS. 25A-25E show only a portion of the introducer hub 518. The channel 580 shown in FIGS. 25A-25E, for example and not by way of limitation, need not be and likely would not be visible by a user from outside the introducer hub 518. However, in order to show the interaction of the dilator 530 with the introducer hub 518, portions of the body 523 of the introducer hub 518 are not shown.

As shown in FIG. 25A, the introducer 510 and the dilator 530 are moved towards each other such that the dilator shaft 538 enters the proximal opening 581 of the introducer hub. In FIG. 25A, the relative longitudinal movement of the dilator 530 with respect to the introducer 510 is indicated by longitudinal movement by the dilator 530 in the first (distal) direction D1 shown in FIG. 25A. However, this is not meant to be limiting, and those skilled in the art will recognize that the longitudinal movement instead can be the introducer 510 moving proximally or a combination of the dilator 530 moving distally and the introducer 510 moving proximally. As shown in FIG. 25A, as the dilator hub 540 approaches the introducer hub 518, the wings 566 of the outer body 560 of the dilator hub 540 are rotationally aligned with the slots 584 in the proximal facing surface 583 of the introducer hub 518. The wings 566 can be aligned with the slots 584 by rotating the outer body 560 of the dilator hub 540.

As shown in FIG. 25B, the dilator 530 continues to be advanced in the first direction D1 such that the wings 566 of the outer body 560 of the dilator hub 540 enter through the slots 584 and into the channel 580. Also, the outer body 560 of dilator hub 540 enters through the opening 581 in the proximal surface 583 of the introducer hub 518 and into central lumen 516. As the wings 566 enter into the channel 580, the outer body 560 of the dilator hub 540 is rotated in the first rotation direction R1 such that the wings 566 are no longer aligned with the slots 584. As the dilator 530 is moved in the first direction D1 and the wings 566 enter the channel 580, the spring 582 contacts a distal surface of the outer body 560. Thus, as the dilator 530 is moved in the first direction D1, the spring 582 provides a force against the dilator 530 in the second direction D2, opposite the first direction D1. Accordingly, once the spring 582 begins acting on the outer body 560, the user must overcome the force of the spring 582 acting in the second direct D2 in order to advance the dilator in the first direction D1. Once the wings 566 are not aligned with the slots 584, if the user releases the dilator 530 *(i.e.,* stops providing a force in the first direction D1) the spring 582 provides a force in the second direction D2 such that the wings 566 abut against the proximal wall 594 of the channel 580. This force acts to lock the positions of the dilator 530 and introducer 510 relative to each other, and hence the positions of the distal tip 522 and the dilator shaft 538 relative to each other.

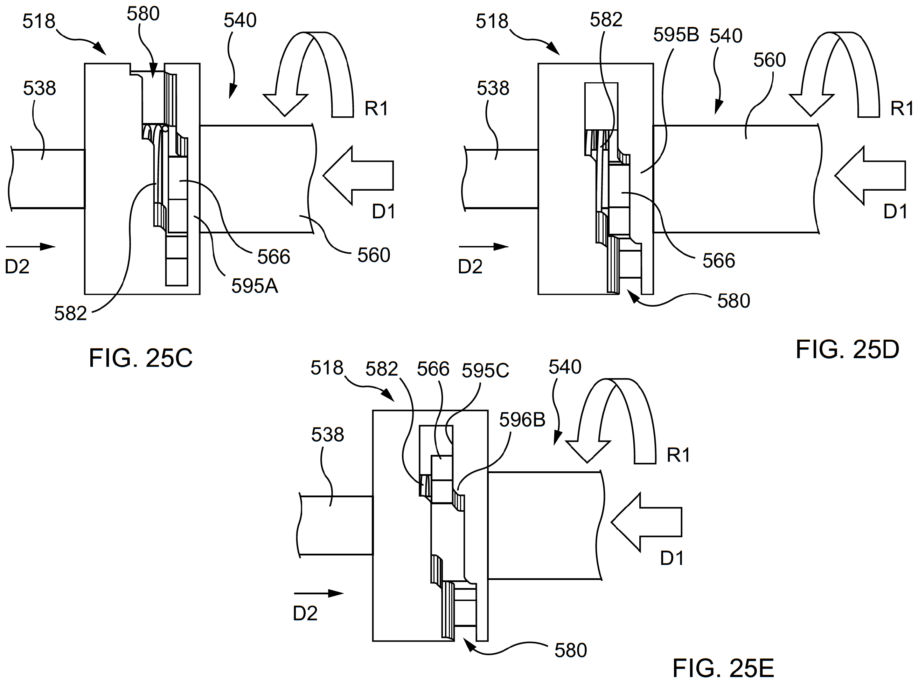

In order to precisely align the distal tip 522 of the introducer sheath 520 with the distal transition portion 550 of the dilator shaft 538, the outer body 560 of the dilator hub 540 can continue to be moved longitudinally in the first direction D1 while being rotated in the first rotational direction R1, as shown in FIGS. 25C, 25D, and 25E. Based on the description above, those skilled in the art will recognize that moving the outer body 560 in the first longitudinal direction D1 also moves the inner body 570 and the dilator shaft 538 in the first longitudinal direction D1. However, rotation of the outer body 560 does not cause the inner body or the dilator shaft 538 to rotate. Further, the description below describes the movement of one of the wings 566 within the channel 580. Those skilled in the art would recognize that the other wing 566 moves in the same manner in the channel 580 not seen in the figures.

As shown in FIG. 25C, the outer body 560 is rotated in the first rotational direction R1 while being pushed in the first longitudinal direction D1 to overcome the force of the spring 582 until the wings 566 are disposed between the distal landing 591B of the distal wall 590 of the channel 580 and the proximal landing 595A of the proximal wall 594 of the channel 580. As the outer body 560 is moved in the first longitudinal direction D1 and rotated in the first rotational direction R1, the location of the dilator shaft 538, and in particular the distal transition portion 550 of the dilator shaft 538, relative to the distal tip 522 of the introducer sheath 520 is observed. If the distal tip 522 is aligned with the distal transition portion 550 with the outer body 560 in the position shown in FIG. 25C, the user may release the outer body 560 and the force of the spring 582 will lock the outer body 560 in place against the proximal landing 595A of the proximal wall 594 of the channel 580. The proximal landing 591A may also include a distal bump (not shown) to prevent the wing 566 from rotating in a second rotational direction opposite the first rotational direction once the wing 566 has been rotated in the first rotational direction R1 and released against the proximal landing 595A. In such an embodiment, if it is desired to rotate the outer body 560 in the second rotational direction opposite the first rotational direction R1, the outer body 560 would be pushed in the first longitudinal direction D1 such that the wing 566 can clear the bump (not shown) to enable the wing 566 to be rotated in the second rotational direction.

If the distal tip 522 of the introducer sheath 520 is observed to be distal of the distal transition portion 550 of the dilator shaft 538, the user continues to push the outer body 560 of the dilator hub 540 in the first longitudinal direction D1 and to rotate the outer body 560 in the first rotational direction R1. Rotation of the outer body 560 causes the wings 566 to rotate relative to the introducer hub 518. As the wing 566 nears the proximal step 596A, the outer body 560 is pushed in the first longitudinal direction D1 to move the wing 566 distally to the proximal landing 595B, as shown in FIG. 25D. This distal movement also moves the inner body 570 and the dilator shaft 538, as described above. If the distal tip 522 is aligned with the distal transition portion 550 with the outer body 560 in the position shown in FIG. 25D, the user may release the outer body 560 and the force of the spring 582 will lock the outer body 560 in place against the proximal landing 595B of the proximal wall 594 of the channel 580. The proximal landing 595B may also include a distal bump (not shown) adjacent the proximal step 596A to prevent the wing 566 from rotating in a second rotational direction opposite the first rotational direction once the wing 566 has been rotated in the first rotational direction R1 and released against the proximal landing 595B. In such an embodiment, if it is desired to rotate the outer body 560 in the second rotational direction opposite the first rotational direction R1, the outer body 560 would be pushed in the first longitudinal direction D1 such that the wing 566 can clear the bump (not shown) to enable the wing 566 to be rotated in the second rotational direction.

If the distal tip 522 of the introducer sheath 520 is observed to be distal of the distal transition portion 550 of the dilator shaft 538, the user continues to push the outer body 560 of the dilator hub 540 in the first longitudinal direction D1 and to rotate the outer body 560 in the first rotational direction R1. Rotation of the outer body 560 causes the wings 566 to rotate relative to the introducer hub 518. As the wing 566 nears the proximal step 596B, the outer body 560 is pushed in the first longitudinal direction D1 to move the wing 566 distally to the proximal landing 595C, as shown in FIG. 25E. This distal movement also moves the inner body 570 and the dilator shaft 538, as described above. As the proximal landing 595C is the distalmost proximal landing, if this position is reached, the distal tip 522 should be aligned with the distal transition portion 550 of the dilator shaft 538, as shown in FIG. 26. The user may release the outer body 560 and the force of the spring 582 will lock the outer body 560 in place against the proximal landing 595B of the proximal wall 594 of the channel 580. The proximal landing 595C may also include a distal bump (not shown) adjacent the proximal step 596B to prevent the wing 566 from rotating in a second rotational direction opposite the first rotational direction once the wing 566 has been rotated in the first rotational direction R1 and released against the proximal landing 595C. In such an embodiment, if it is desired to rotate the outer body 560 in the second rotational direction opposite the first rotational direction R1, the outer body 560 would be pushed in the first longitudinal direction D1 such that the wing 566 can clear the bump (not shown) to enable the wing 566 to be rotated in the second rotational direction.

It would be understood by those skilled in the art that the embodiment shown in FIGS. 20-25E may be modified without departing from the spirit of the invention. For example, and not by way of limitation, there may be more or fewer proximal landings, distal landings, proximal steps, and distal steps and they may be different sizes, thereby varying the quantity of positions of the dilator 530 relative to the introducer 510 and the incremental longitudinal movement of the dilator 530 when the wings 566 step from one landing to the next landing.

Referring to FIG. 26, distal portions of the introducer sheath 120, 420, 520 and the dilator shaft 138, 438, 538 are shown. As can be seen, the fine adjustment of the relative positions of the dilator 130, 439, 530 and the introducer 110, 410, 510 relative to each other enables accurate positioning of the distal tip 122, 422, 522 of the introducer sheath 120, 420, 520. Such accurate positioning enables the distal transition portion 150, 450, 550 of the dilator shaft 138, 438, 538 to be shorter than the embodiment of FIG. 5. Accordingly, the length L1 of the distal transition portion 150, 450, 550 may be in the range of about 0.0 mm (no offset such that the distal transition portion 150, 450, 550 is perpendicular to the middle portion 146, 246, 546 of the dilator shaft 138, 438, 538) to about 10 mm.

In some embodiments, the dilator shaft 138, 438, 538 may include a marker 153 (shown in FIGS. 11 and 13) indicating the desired position of the dilator shaft 138, 438, 538 relative to the distal tip 122, 422, 422 of the introducer sheath 120, 420, 520. Although shown in FIGS. 11 and 13, the marker 153 may also be included in the embodiment shown in FIG. 26. The marker 153 may be a line disposed on distal transition portion 150, and may extend partially or entirely around the circumference of the distal transition portion 150. The marker 153 may be laser marked, pad printed, or otherwise applied to the distal transition portion 150. The marker 153 enables the user (e.g. physician) to ensure that the introducer and the dilator are properly positioned prior to insertion into the patient, thereby minimizing insertion forces required and potential trauma to the patient.

As used herein, the terms "generally" and "substantially" mean approximately. When used to describe angles such as "substantially parallel" or "substantially perpendicular" the term "substantially" means within 10 degrees of the angle. When used to describe shapes such as "substantially" or "generally" cylindrical or "substantially" or "generally" tube-shaped or "generally" or "substantially" conical, the terms mean that the shape would appear cylindrical or tube-shaped or conical to a person of ordinary skill in the art viewing the shape with a naked eye.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. It should be understood that use of the term "about" also includes the specifically recited number of value.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components.

The following examples are illustrative of the techniques described herein.

Example 1. An introducer system comprising: an introducer including an introducer hub, an introducer sheath, and an introducer tip, the introducer including an introducer proximal end, and introducer distal end, and an introducer central lumen extending from the introducer proximal end to the introducer distal end; a dilator including a dilator hub and a dilator shaft; and a coupling arrangement configured to couple the dilator to the introducer, wherein the coupling arrangement is configured to selectively enable rotation of the dilator relative to the introducer to longitudinally translate the dilator relative to the introducer.

Example 2. The introducer system of Example 1, wherein the coupling arrangement is a coupling ring.

Example 3. The introducer system of Example 2, wherein the coupling ring is coupled to the dilator of the introducer system.

Example 4. The introducer system of Example 2, wherein the coupling ring is coupled to the introducer of the introducer system.

Example 5. The introducer system of Example 2, wherein the coupling ring comprises a distal section configured to couple the coupling ring to the introducer hub.

Example 6. The introducer system of Example 5, wherein the dilator hub comprises a distal portion including a first thread on an outer surface and the coupling ring comprises a middle section including a second thread on an inner surface, wherein the first thread of the distal portion of the dilator hub threadably engages the second thread of the middle section of the coupling ring to enable longitudinal translation of the dilator relative to the coupling ring when the dilator hub is rotated relative to the coupling ring.

Example 7. The introducer system of Example 6, wherein in a first configuration, the coupling ring is coupled to the introducer hub, the dilator is disposed through the introducer, a distal end of the dilator hub abuts a distal end of the distal end of the middle section of the coupling ring, and a distal end of the dilator shaft extends distally past the introducer tip, and wherein rotation of the dilator hub in a first direction relative to the coupling ring longitudinally translates the dilator proximally relative to introducer to a second configuration.

Example 8. The introducer system of Example 2, further comprising a lock configured to selectively transition the dilator and the coupling ring between a locked configuration in which the dilator hub is prevented from rotating relative to the coupling ring and an unlocked configuration in which the dilator hub is enabled to rotate relative to the coupling ring.

Example 9. The introducer system of Example 8, wherein the lock comprises: a tab including a first end and a second end; an actuator coupled to the second end of the tab and configured to be selectively actuated by a user to transition the lock assembly between the locked configuration and the unlocked configuration; and a biasing element configured to urge the lock assembly to the locked configuration.

Example 10. The introducer system of Example 9, wherein the coupling ring comprises a plurality of slots, wherein in the locked configuration the tab of the lock is disposed within one of the plurality of slots to prevent rotation of the dilator hub relative to the coupling ring, and wherein in the unlocked configuration the tab is not disposed in any of the plurality of slots such that the dilator hub is free to rotate relative to the coupling ring.

Example 11. The introducer system of Example 1, wherein: the dilator hub comprises an outer body and an inner body disposed within the outer body, wherein the outer body is rotatably coupled to the inner body such that rotation of the outer body does not rotate the inner body and longitudinal translation of the outer body causes longitudinal translation of the inner body, wherein the dilator shaft is coupled to the inner body such that movement of the inner body causes corresponding movement of the dilator shaft; the introducer hub comprises a proximal extension extending proximally from a body of the introducer hub; and the coupling arrangement comprises a first thread and a second thread coupling the outer body to the proximal extension, wherein the first thread and the second thread convert rotation of the outer body to longitudinal translation of the dilator hub relative to the introducer hub, and hence longitudinal translation of the dilator shaft relative to the introducer sheath.

Example 12. The introducer system of Example 11, wherein the proximal extension is a cylindrical extension defining a portion of a central lumen of the introducer, wherein the proximal extension includes the first thread on an outer surface thereof; wherein the outer body includes a distal portion, wherein the second thread is disposed on an inner surface of the distal portion.

Example 13. The introducer system of Example 11, wherein the outer body includes a tab extending radially inward and circumferentially around an inner surface of the outer body, and wherein the inner body includes a recess extending radially inward and circumferentially around an outer surface of the inner body, wherein the tab and the recess rotatably couple the outer body to the inner body.

Example 14. The introducer system of Example 11, further comprising a lock configured to lock the relative positions of the introducer and the dilator.

Example 15. The introducer system of Example 14, wherein the lock comprises a slider disposed on the introducer hub and a ridged distal end of the outer body of the dilator hub, wherein the slider comprises a shaped end, wherein in an unlocked configuration, the shaped end of the slider is spaced from the ridged distal end of the outer body, and wherein in a locked configuration, the shaped end of the slider engages the ridged distal end of the outer body.

Example 16. The introducer system of Example 1, wherein the coupling arrangement comprises tabs of the dilator hub interacting with a stepped channel of the introducer hub such that rotation of the dilator hub converts to longitudinal translation of the dilator relative to the introducer.

Example 17. The introducer system of Example 16, wherein: the dilator hub comprises an outer body and an inner body disposed within the outer body, wherein the outer body is rotatably coupled to the inner body such that rotation of the outer body does not rotate the inner body and longitudinal translation of the outer body causes longitudinal translation of the inner body, wherein the dilator shaft is coupled to the inner body such that movement of the inner body causes corresponding movement of the dilator shaft, wherein the dilator hub comprises tabs at a distal end thereof; and the tabs extend radially outward from a distal portion of the outer body.

Example 18. The introducer system of Example 17, further comprising a lock configured to lock the relative positions of the introducer and the dilator.

Example 19. The introducer system of Example 18, wherein the lock comprises a spring disposed within the introducer hub, wherein the spring is configured to act on the outer body of the dilator hub to press the tabs against a wall of the stepped channel to prevent relative movement between the dilator and the introducer.

Example 20. A method of adjusting a relative longitudinal position of an introducer and a dilator of an introducer system comprising: disposing the dilator through the introducer; coupling a dilator hub of the dilator with an introducer hub to the introducer; rotating the dilator hub to relative to the introducer hub to longitudinally translate the dilator relative to the introducer; aligning a distal tip of an introducer sheath of the introducer with a distal transition portion of a dilator shaft of the dilator such that the introducer and the dilator are in an aligned configuration; and with the dilator and the introducer in the aligned configuration, transitioning the dilator and the introducer to a locked configuration to prevent rotation of the dilator hub relative to the introducer hub.

## Claims

1. An introducer system comprising:
an introducer including an introducer hub, an introducer sheath, and an introducer tip, the introducer including an introducer proximal end, and introducer distal end, and an introducer central lumen extending from the introducer proximal end to the introducer distal end;
a dilator including a dilator hub and a dilator shaft; and
a coupling arrangement configured to couple the dilator to the introducer, wherein the coupling arrangement is configured to selectively enable rotation of the dilator relative to the introducer to longitudinally translate the dilator relative to the introducer.

2. The introducer system of claim 1, wherein the coupling arrangement is a coupling ring.

3. The introducer system of claim 2, wherein the coupling ring is coupled to the dilator of the introducer system or the introducer of the introducer system.

4. The introducer system of claim 2 or claim 3, wherein the coupling ring comprises a distal section configured to couple the coupling ring to the introducer hub.

5. The introducer system of any one of claims 2 to 4, wherein the dilator hub comprises a distal portion including a first thread on an outer surface and the coupling ring comprises a middle section including a second thread on an inner surface, wherein the first thread of the distal portion of the dilator hub threadably engages the second thread of the middle section of the coupling ring to enable longitudinal translation of the dilator relative to the coupling ring when the dilator hub is rotated relative to the coupling ring.

6. The introducer system of any one of claims 2 to 5, further comprising a lock assembly configured to selectively transition the dilator and the coupling ring between a locked configuration in which the dilator hub is prevented from rotating relative to the coupling ring and an unlocked configuration in which the dilator hub is enabled to rotate relative to the coupling ring.

7. The introducer system of claim 6, wherein the lock assembly comprises:
a tab including a first end and a second end;
an actuator coupled to the second end of the tab and configured to be selectively actuated by a user to transition the lock assembly between the locked configuration and the unlocked configuration; and
a biasing element configured to urge the lock assembly to the locked configuration.

8. The introducer system of claim 7, wherein the coupling ring comprises a plurality of slots, wherein in the locked configuration the tab of the lock assembly is disposed within one of the plurality of slots to prevent rotation of the dilator hub relative to the coupling ring, and wherein in the unlocked configuration the tab is not disposed in any of the plurality of slots such that the dilator hub is free to rotate relative to the coupling ring.

9. The introducer system of claim 1, wherein:
the dilator hub comprises an outer body and an inner body disposed within the outer body, wherein the outer body is rotatably coupled to the inner body such that rotation of the outer body does not rotate the inner body and longitudinal translation of the outer body causes longitudinal translation of the inner body, wherein the dilator shaft is coupled to the inner body such that movement of the inner body causes corresponding movement of the dilator shaft;
the introducer hub comprises a proximal extension extending proximally from a body of the introducer hub; and
the coupling arrangement comprises a first thread and a second thread coupling the outer body to the proximal extension, wherein the first thread and the second thread convert rotation of the outer body to longitudinal translation of the dilator hub relative to the introducer hub, and hence longitudinal translation of the dilator shaft relative to the introducer sheath.

10. The introducer system of claim 9,
wherein the proximal extension is a cylindrical extension defining a portion of a central lumen of the introducer, wherein the proximal extension includes the first thread on an outer surface thereof; and
wherein the outer body includes a distal portion, wherein the second thread is disposed on an inner surface of the distal portion.

11. The introducer system of claim 1, wherein the coupling arrangement comprises tabs of the dilator hub interacting with a stepped channel of the introducer hub such that rotation of the dilator hub converts to longitudinal translation of the dilator relative to the introducer.

12. The introducer system of claim 11, wherein:
the dilator hub comprises an outer body and an inner body disposed within the outer body, wherein the outer body is rotatably coupled to the inner body such that rotation of the outer body does not rotate the inner body and longitudinal translation of the outer body causes longitudinal translation of the inner body, wherein the dilator shaft is coupled to the inner body such that movement of the inner body causes corresponding movement of the dilator shaft, wherein the dilator hub comprises tabs at a distal end thereof; and
the tabs extend radially outward from a distal portion of the outer body.

13. The introducer system of any one of claims 9 to 12, further comprising a lock configured to lock the relative positions of the introducer and the dilator.

14. The introducer system of claim 11 or claim 12, further comprising a lock configured to lock the relative positions of the introducer and the dilator, wherein the lock comprises a spring disposed within the introducer hub, wherein the spring is configured to act on the outer body of the dilator hub to press the tabs against a wall of the stepped channel to prevent relative movement between the dilator and the introducer.

15. The introducer system of claim 9 or claim 10, further comprising a lock configured to lock the relative positions of the introducer and the dilator, wherein the lock comprises a slider disposed on the introducer hub and a ridged distal end of the outer body of the dilator hub, wherein the slider comprises a shaped end, wherein in an unlocked configuration, the shaped end of the slider is spaced from the ridged distal end of the outer body, and wherein in a locked configuration, the shaped end of the slider engages the ridged distal end of the outer body.
